# EUROPEAN PATENT SPECIFICATION

(11) **EP 4 376 808 B1**
(45) Date of publication and mention of the grant of the patent: **06.05.2026**
(21) Application number: 22738212.4
(22) Date of filing: 13.06.2022
(51) Int. Cl.: A61K 45/06, A61K 31/4184, A61K 9/08, A61K 47/10, A61K 47/12, A61P 25/08

(54) **CLEMIZOLE FORMULATION**
CLEMISOLFORMULIERUNG
FORMULATION DE CLÉMIZOLE

(30) Priority: 30.07.2021 US 202163227778 P
(43) Date of publication of application: 05.06.2024
(60) Divisional application: 26168187.8
(73) Proprietor: Epygenix Therapeutics, Inc., Paramus, New Jersey 07652 (US)
(72) Inventor: LEE, Hahn-Jun, Paramus, New Jersey 07652 (US)
(74) Representative: Nederlandsch Octrooibureau
(86) International application number: PCT/US2022/033283
(87) International publication number: WO 2023/009228

(56) References cited:
- EP-A1- 0 436 729
- WO-A1-2015/026849
- WO-A1-2016/067024
- WO-A1-2016/138138
- WO-A1-2019/175703
- FR-A1- 2 218 086
- GB-A- 2 577 363

## Description

### FIELD OF INVENTION

The disclosure herein relates to providing an oral formulation of clemizole HCl that is soluble and stable over time, and the methods for preparing such oral clemizole HCl formulations.

### BACKGROUND

Clemizole HCl is the hydrochloride salt form of clemizole, a first-generation histamine receptor (H1) antagonist, a class of drugs that has been shown to have anti-allergic activities. But clemizole also acts as an anti-epileptic agent, inhibiting behavioral and electrographic seizure activity. This anti-seizure-like activity is thought to be regulated in part by clemizole's role in modulating serotonin (5-HT) receptor signaling.

Serotonin (5-HT) receptor signaling has also been shown to play a role in anti-seizure activity, including Dravet syndrome, which is a severe childhood epilepsy syndrome with early onset seizures, severe cognitive defects, delayed language, and delayed motor development. Attenuation of seizure activity can be obtained with certain treatments, but a common widespread, effective treatment is still needed.

### SUMMARY

In an aspect, the invention relates to a liquid pharmaceutical formulation comprising, consisting essentially of, or consisting of clemizole HCl, a solvent, a preservative, glycerol, a flavoring agent, a sweetener, and a solubilizer. In an aspect, the clemizole HCl formulation is stable and retains its solubility over at least certain prescribed time periods. The clemizole HCl has a solubility of 1 mg/mL - 30 mg/mL in solution. In an aspect, the invention relates to a liquid pharmaceutical formulation having a pH value of 4.5, comprising, consisting essentially of, or consisting of clemizole HCl, a pH 4.5 citrate buffer, glycerol, a sweetener, a flavoring agent, a solubilizer (polyethylene glycol (PEG)-400), and a preservative (methyl parahydroxybenzoate and ethyl parahydroxybenzoate). In an aspect, the clemizole HCl and the preservative are stable for up to 36 months.

In an aspect, the invention relates to a method for generating a clemizole HCl liquid pharmaceutical formulation, comprising, consisting essentially of, or consisting of dissolving at least one preservative in a solution comprising at least one of a solvent, a solubilizer, or a combined solvent-solubilizer solution; dissolving clemizole HCl in a solution comprising at least one of the solvent, the solubilizer, or the combined solvent-solubilizer solution; dissolving a plurality of excipients in a solution comprising at least one of a solvent, a solubilizer, or a combined solvent-solubilizer solution; adding glycerol; and adjusting the pH, such that the final concentration of clemizole HCl in the liquid pharmaceutical composition ranges from 1 mg/mL - 30 mg/mL and the formulation is soluble and stable over time. In an aspect, the clemizole HCl and the preservative are stable for up to 36 months. In an aspect, the invention relates to a method for generating a clemizole HCl liquid pharmaceutical formulation, comprising, consisting essentially of, or consisting of dispensing PEG400 into a beaker, adding ethyl parahydroxybenzoate, adding methyl parahydroxybenzoate (ensuring that the first preservative is fully dissolved before adding the second preservative), adding pH 4.5 citrate buffer to a second beaker, adding clemizole HCl to the second beaker containing the buffer and mixing until fully dissolved, adding the solubilizer containing preservatives to the second beaker containing buffer and clemizole HCl while stirring, adding sweetener, adding a flavoring following full dissolution of the sweetener, adding glycerol and ensuring the mixture is homogeneous, measuring the pH and adjusting the pH to 4.5 +/- 0.5, and bringing to volume.

Any references in the description to methods of treatment refer to the compounds, pharmaceutical compositions and medicaments of the present disclosure for use in a method for treatment of the human (or animal) body by therapy (or for diagnosis). In an aspect, the invention relates to a method of treating a subject having an epilepsy disorder, comprising administering a liquid pharmaceutical formulation comprising clemizole HCl. In an aspect, the formulation has a concentration range of clemizole HCl of 1 mg/mL - 30 mg/mL. In an aspect, the clemizole HCl liquid pharmaceutical formulation is prepared by a method comprising, consisting essentially of, or consisting of dissolving at least one preservative in a solution comprising at least one of a solvent, a solubilizer, or a combined solvent-solubilizer solution; dissolving clemizole HCl in a solution comprising at least one of the solvent, the solubilizer, or the combined solvent-solubilizer solution; dissolving a plurality of excipients in a solution comprising at least one of a solvent, a solubilizer, or a combined solvent-solubilizer solution; adding glycerol; and adjusting the pH. In an aspect, the epilepsy disorder may be Dravet syndrome, benign Rolandic epilepsy, frontal lobe epilepsy, infantile spasm, juvenile myoclonic epilepsy (JME), juvenile absence epilepsy, childhood absence epilepsy (e.g., pyknolepsy), febrile seizures, progressive myoclonus epilepsy of Lafora, Lennox-Gastaut syndrome, Landau-Kleffner syndrome, Generalized Epilepsy with Febrile Seizures (GEFS+), Severe Myoclonic Epilepsy of Infancy (SMEI), Benign Neonatal Familial Conversions (BNFC), West Syndrome, Ohtahara Syndrome, early myoclonic encephalopathies, migration partial epilepsy, infantile epileptic encephalopathies, Tuberous Sclerosis Complex (TSC), focal cortical dysplasia, Type I Lissencephaly, Miller-Dieker Syndrome, Angelman's syndrome, Fragile X syndrome, epilepsy in autism spectrum disorder, subcortical band heterotopia, Walker-Warburg syndrome, Alzheimer's disease, posttraumatic epilepsy, progressive myoclonus epilepsies, reflex epilepsy, Rasmussen's syndrome, temporal lobe epilepsy, limbic epilepsy, status epilepticus, abdominal epilepsy, massive bilateral myoclonus catamenial epilepsy, Jaksonian seizure disorder, Unverricht-Lundborg disease, or photosensitive epilepsy.

### DETAILED DESCRIPTION

An embodiment includes a liquid pharmaceutical composition. The liquid pharmaceutical composition may be for the treatment of an epilepsy disorder. In an embodiment, the epilepsy disorder may be a pediatric epilepsy disorder. In an embodiment, the epilepsy disorder may be Dravet syndrome, benign Rolandic epilepsy, frontal lobe epilepsy, infantile spasm, juvenile myoclonic epilepsy (JME), juvenile absence epilepsy, childhood absence epilepsy (e.g., pyknolepsy), febrile seizures, progressive myoclonus epilepsy of Lafora, Lennox-Gastaut syndrome, Landau-Kleffner syndrome, Generalized Epilepsy with Febrile Seizures (GEFS+), Severe Myoclonic Epilepsy of Infancy (SMEI), Benign Neonatal Familial Conversions (BNFC), West Syndrome, Ohtahara Syndrome, early myoclonic encephalopathies, migration partial epilepsy, infantile epileptic encephalopathies, Tuberous Sclerosis Complex (TSC), focal cortical dysplasia, Type I Lissencephaly, Miller-Dieker Syndrome, Angelman's syndrome, Fragile X syndrome, epilepsy in autism spectrum disorder, subcortical band heterotopia, Walker-Warburg syndrome, Alzheimer's disease, posttraumatic epilepsy, progressive myoclonus epilepsies, reflex epilepsy, Rasmussen's syndrome, temporal lobe epilepsy, limbic epilepsy, status epilepticus, abdominal epilepsy, massive bilateral myoclonus catamenial epilepsy, Jaksonian seizure disorder, Unverricht-Lundborg disease, or photosensitive epilepsy. The liquid pharmaceutical composition may comprise, consist essentially of, or consist of clemizole HCl, a solvent, a preservative, and a co-solvent. The liquid pharmaceutical composition may have a pH of 4.5. The liquid pharmaceutical composition may have a pH of 4.5 +/- 0.5.

The clemizole HCl may be present in the liquid pharmaceutical composition at 1 mg/mL, 1.125 mg/mL, 2.5 mg/mL, 5.0 mg/mL, 7.5 mg/mL, 10 mg/mL, 15 mg/mL, 20 mg/mL, 25 mg/mL, or 30 mg/mL. In an embodiment, the clemizole HCl may be present at 1 mg/mL to 2.5 mg/mL, 2.5 mg/mL to 5.0 mg/mL, 5.0 mg/mL to 7.5 mg/mL, 7.5 mg/mL to 10 mg/mL, 10 mg/mL to 15 mg/mL, 15 mg/mL to 20 mg/mL, 20 mg/mL to 25 mg/mL, 25 mg/mL to 30 mg/mL, or any sub-range in between any of these concentration ranges. In an embodiment, the clemizole HCl may be present from 0.5 mg/mL increments in the range of 1 mg/mL to 30 mg/mL (that is, the concentration in the liquid pharmaceutical composition may be 1 mg/mL, 1.5 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 10.5 mg/mL, 11 mg/mL. 11.5 mg/mL, 12 mg/mL, 12.5 mg/mL, 13 mg/mL, 13.5 mg/mL, 14 mg/mL, 14.5 mg/mL, 15 mg/mL, 15.5 mg/mL, 16 mg/mL, 16.5 mg/mL, 17 mg/mL, 17.5 mg/mL, 18 mg/mL, 18.5 mg/mL, 19 mg/mL, 19.5 mg/mL, 20 mg/mL, 20.5 mg/mL, 21 mg/mL, 21.5 mg/mL, 22 mg/mL, 22.5 mg/mL, 23 mg/mL, 23.5 mg/mL, 24 mg/mL, 24.5 mg/mL, 25 mg/mL, 25.5 mg/mL, 26 mg/mL, 26. 5 mg/mL, 27 mg/mL, 27.5 mg/mL, 28 mg/mL, 28.5 mg/mL, 29 mg/mL, 29.5 mg/mL, or 30 mg/mL). In an embodiment, the clemizole HCl may be present from 1 mg/mL to 30 mg/mL, or in a sub-range thereof. The sub-range low endpoint may be selected from 1 mg/mL, 1.5 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 10.5 mg/mL, 11 mg/mL. 11.5 mg/mL, 12 mg/mL, 12.5 mg/mL, 13 mg/mL, 13.5 mg/mL, 14 mg/mL, 14.5 mg/mL, 15 mg/mL, 15.5 mg/mL, 16 mg/mL, 16.5 mg/mL, 17 mg/mL, 17.5 mg/mL, 18 mg/mL, 18.5 mg/mL, 19 mg/mL, 19.5 mg/mL, 20 mg/mL, 20.5 mg/mL, 21 mg/mL, 21.5 mg/mL, 22 mg/mL, 22.5 mg/mL, 23 mg/mL, 23.5 mg/mL, 24 mg/mL, 24.5 mg/mL, 25 mg/mL, 25.5 mg/mL, 26 mg/mL, 26. 5 mg/mL, 27 mg/mL, 27.5 mg/mL, 28 mg/mL, 28.5 mg/mL, 29 mg/mL, or 29.5 mg/mL, while the sub-range high endpoint may be selected from 1.5 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 10.5 mg/mL, 11 mg/mL. 11.5 mg/mL, 12 mg/mL, 12.5 mg/mL, 13 mg/mL, 13.5 mg/mL, 14 mg/mL, 14.5 mg/mL, 15 mg/mL, 15.5 mg/mL, 16 mg/mL, 16.5 mg/mL, 17 mg/mL, 17.5 mg/mL, 18 mg/mL, 18.5 mg/mL, 19 mg/mL, 19.5 mg/mL, 20 mg/mL, 20.5 mg/mL, 21 mg/mL, 21.5 mg/mL, 22 mg/mL, 22.5 mg/mL, 23 mg/mL, 23.5 mg/mL, 24 mg/mL, 24.5 mg/mL, 25 mg/mL, 25.5 mg/mL, 26 mg/mL, 26. 5 mg/mL, 27 mg/mL, 27.5 mg/mL, 28 mg/mL, 28.5 mg/mL, 29 mg/mL, 29.5 mg/mL, or 30.0 mg/mL, where the high endpoint selected is higher than the low endpoint selected. In an embodiment, the clemizole HCl may be present at 5.0 mg/mL, 10 mg/mL, or 15 mg/mL. In an embodiment, the clemizole HCl may be present at 5.0 mg/mL.

The solvent may be an acidic solution. The solvent may comprise, consist essentially of, or consist of a citrate buffer having an acidic pH, or a 0.1 M HCl solution. In an embodiment, the solvent may be a citrate buffer having a pH of 4.5. In an embodiment, the citrate buffer may have a pH of 4.5 +/- 0.5. In an embodiment the solvent may comprise, consist essentially of, or consist of a co-solvent.

The solubility of clemizole HCl in the solvent and/or solvent-co-solvent solution may be in the range of 1 mg/mL to 30 mg/mL. In an embodiment, the solubility of clemizole HCl in the solvent and/or solvent-co-solvent solution may be selected from 1 mg/mL, 1.5 mg/mL, 2.5 mg/mL to 5.0 mg/mL, 5.0 mg/mL to 7.5 mg/mL, 7.5 mg/mL to 10 mg/mL, 10 mg/mL to 15 mg/mL, 15 mg/mL to 20 mg/mL, 20 mg/mL to 25 mg/mL, 25 mg/mL to 30 mg/mL, or any sub-range in between any of these concentration ranges. In an embodiment, the solubility of clemizole HCl in the solvent and/or solvent-co-solvent solution may be in 0.5 mg/mL increments in the range of 1 mg/mL to 30 mg/mL (that is, the solubility in the solvent and/or co-solvent may be 1 mg/mL, 1.5 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 10.5 mg/mL, 11 mg/mL. 11.5 mg/mL, 12 mg/mL, 12.5 mg/mL, 13 mg/mL, 13.5 mg/mL, 14 mg/mL, 14.5 mg/mL, 15 mg/mL, 15.5 mg/mL, 16 mg/mL, 16.5 mg/mL, 17 mg/mL, 17.5 mg/mL, 18 mg/mL, 18.5 mg/mL, 19 mg/mL, 19.5 mg/mL, 20 mg/mL, 20.5 mg/mL, 21 mg/mL, 21.5 mg/mL, 22 mg/mL, 22.5 mg/mL, 23 mg/mL, 23.5 mg/mL, 24 mg/mL, 24.5 mg/mL, 25 mg/mL, 25.5 mg/mL, 26 mg/mL, 26. 5 mg/mL, 27 mg/mL, 27.5 mg/mL, 28 mg/mL, 28.5 mg/mL, 29 mg/mL, 29.5 mg/mL, or 30 mg/mL). In an embodiment, the solubility of clemizole HCl in the solvent and/or solvent-co-solvent solution may be in 1 mg/mL to 30 mg/mL, or in a sub-range thereof. The sub-range low endpoint may be selected from 1 mg/mL, 1.5 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 10.5 mg/mL, 11 mg/mL. 11.5 mg/mL, 12 mg/mL, 12.5 mg/mL, 13 mg/mL, 13.5 mg/mL, 14 mg/mL, 14.5 mg/mL, 15 mg/mL, 15.5 mg/mL, 16 mg/mL, 16.5 mg/mL, 17 mg/mL, 17.5 mg/mL, 18 mg/mL, 18.5 mg/mL, 19 mg/mL, 19.5 mg/mL, 20 mg/mL, 20.5 mg/mL, 21 mg/mL, 21.5 mg/mL, 22 mg/mL, 22.5 mg/mL, 23 mg/mL, 23.5 mg/mL, 24 mg/mL, 24.5 mg/mL, 25 mg/mL, 25.5 mg/mL, 26 mg/mL, 26. 5 mg/mL, 27 mg/mL, 27.5 mg/mL, 28 mg/mL, 28.5 mg/mL, 29 mg/mL, or 29.5 mg/mL, while the sub-range high endpoint may be selected from 1.5 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 10.5 mg/mL, 11 mg/mL. 11.5 mg/mL, 12 mg/mL, 12.5 mg/mL, 13 mg/mL, 13.5 mg/mL, 14 mg/mL, 14.5 mg/mL, 15 mg/mL, 15.5 mg/mL, 16 mg/mL, 16.5 mg/mL, 17 mg/mL, 17.5 mg/mL, 18 mg/mL, 18.5 mg/mL, 19 mg/mL, 19.5 mg/mL, 20 mg/mL, 20.5 mg/mL, 21 mg/mL, 21.5 mg/mL, 22 mg/mL, 22.5 mg/mL, 23 mg/mL, 23.5 mg/mL, 24 mg/mL, 24.5 mg/mL, 25 mg/mL, 25.5 mg/mL, 26 mg/mL, 26. 5 mg/mL, 27 mg/mL, 27.5 mg/mL, 28 mg/mL, 28.5 mg/mL, 29 mg/mL, 29.5 mg/mL, or 30 mg/mL, where the high endpoint selected is higher than the low endpoint selected. In an embodiment, the solubility of clemizole HCl in the solvent and/or solvent-co-solvent solution may be 5 mg/mL, 10 mg/mL, or 15 mg/mL. In an embodiment, the solubility of clemizole HCl in the solvent and/or solvent-co-solvent solution may be 5 mg/mL.

In an embodiment the co-solvent may comprise, consist essentially of, or consist of glycerol, Cremophor ELP, Cremophor RH40, Polysorbate 20, Polysorbate 80, tocopheryl polyethylene glycol succinate (TPGS), Solutol HS15, polyethylene glycol (PEG)-400, propylene glycol, 2-hydroxypropyl-beta-cyclodextrin, sodium lauryl sulphate (10% w/w in water) and sodium docusate (10% w/w in water), or any other compatible co-solvent capable of forming a co-solution having a solubility as described in the above-referenced paragraph. In an embodiment the co-solvent may be glycerol.

In an embodiment, the solvent may be a citrate buffer having a pH of 4.5 and the co-solvent may be glycerol. In an embodiment, the citrate buffer may have a pH of 4.5 +/- 0.5. In an embodiment the ratio of pH 4.5 citrate buffer to glycerol may be in the range of 99% and 1%, respectively, to 70% and 30%, respectively. In an embodiment, the ratio of pH 4.5 citrate buffer to glycerol may be in 0.5% increments in the range of 70% to 99% for the pH 4.5 citrate buffer and 1% to 30% for the glycerol, respectively. In an embodiment the ratio of pH 4.5 citrate buffer to glycerol may be in 99% and 1%, 98.5% and 1.5%, 98% and 2 %, 97.5% and 2.5%, 97% and 3%, 96.5% and 3.5%, 96% and 4%, 95.5% and 4.5%, 95% and 5%, 94.5% and 5.5%, 94% and 6%, 93.5% and 6.5%, 93% and 7%, 92.5% and 7.5%, 92% and 8%, 91.5% and 8.5%, 91% and 9%, 90.5% and 9.5%, 90% and 10%, 89.5% and 10.5%, 89% and 11%, 88.5% and 11.5%, 88% and 12%, 87.5% and 12.5%, 87% and 13%, 86.5% and 13.5%, 86% and 14%, 85.5% and 14.5%, 85% and 15%, 84.5% and 15.5%, 84% and 16%, 83.5% and 16.5%, 83% and 17%, 82.5% and 17.5%, 82% and 18%, 81.5% and 18.5%, 81% and 19%, 80.5% and 19.5%, 80% and 20%, 79.5% and 20.5%, 79% and 21%, 78.5% and 21.5%, 78% and 22%, 77.5% and 22.5%, 77% and 23%, 76.5% and 23.5%, 76% and 24%, 75.5% and 24.5%, 75% and 25%, 74.5% and 25.5%, 74% and 26%, 73.5% and 26.5%, 73% and 27%, 72.5% and 27.5%, 72% and 28%, 71.5% and 28.5%, 71% and 29%, 70.5% and 29.5%, and 70% and 30%, respectively In an embodiment the ratio of pH 4.5 citrate buffer to glycerol may be 90% and 10%, respectively; 80% and 20%, respectively; or 70% and 30%, respectively. In an embodiment the ratio of pH 4.5 citrate buffer to glycerol may be 80% and 20%, respectively.

In an embodiment, the viscosity of the solvent-glycerol solution is such that there is at least a moderate continuous flow to a continuous flow of the solution through test oral dosing syringes. The test oral dosing syringes may be an Adelphi "Elm Tip," code 706100 and an Adelphi Flat Tip, code 90500. In an embodiment, the solvent is pH 4.5 citrate buffer.

In an embodiment, the solubility of clemizole HCl in the pH 4.5 citrate buffer-glycerol solution may be in the range of 1 mg/mL to 30 mg/mL. In an embodiment, the solubility of clemizole HCl in the pH 4.5 citrate buffer-glycerol solution may be 1 mg/mL to 1.5 mg/mL, 2 mg/mL. 2/5 mg/mL to 5.0 mg/mL, 5.0 mg/mL to 7.5 mg/mL, 7.5 mg/mL to 10 mg/mL, 10 mg/mL to 15 mg/mL, 15 mg/mL to 20 mg/mL, 20 mg/mL to 25 mg/mL, 25 mg/mL to 30 mg/mL, or any sub-range in between any of these concentration ranges. In an embodiment, the solubility of clemizole HCl in the pH 4.5 citrate buffer-glycerol solution may be in 0.5 mg/mL increments in the range of 1 mg/mL to 30 mg/mL (that is, the solubility in the solvent and/or co-solvent may be 1 mg/mL, 1.5 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 10.5 mg/mL, 11 mg/mL. 11.5 mg/mL, 12 mg/mL, 12.5 mg/mL, 13 mg/mL, 13.5 mg/mL, 14 mg/mL, 14.5 mg/mL, 15 mg/mL, 15.5 mg/mL, 16 mg/mL, 16.5 mg/mL, 17 mg/mL, 17.5 mg/mL, 18 mg/mL, 18.5 mg/mL, 19 mg/mL, 19.5 mg/mL, 20 mg/mL, 20.5 mg/mL, 21 mg/mL, 21.5 mg/mL, 22 mg/mL, 22.5 mg/mL, 23 mg/mL, 23.5 mg/mL, 24 mg/mL, 24.5 mg/mL, 25 mg/mL, 25.5 mg/mL, 26 mg/mL, 26. 5 mg/mL, 27 mg/mL, 27.5 mg/mL, 28 mg/mL, 28.5 mg/mL, 29 mg/mL, 29.5 mg/mL, or 30 mg/mL). In an embodiment, the solubility of clemizole HCl in the pH 4.5 citrate buffer-glycerol solution may be 1 mg/mL to 30 mg/mL, or in a sub-range thereof. The sub-range low endpoint may be selected from 1 mg/mL, 1.5 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 10.5 mg/mL, 11 mg/mL. 11.5 mg/mL, 12 mg/mL, 12.5 mg/mL, 13 mg/mL, 13.5 mg/mL, 14 mg/mL, 14.5 mg/mL, 15 mg/mL, 15.5 mg/mL, 16 mg/mL, 16.5 mg/mL, 17 mg/mL, 17.5 mg/mL, 18 mg/mL, 18.5 mg/mL, 19 mg/mL, 19.5 mg/mL, 20 mg/mL, 20.5 mg/mL, 21 mg/mL, 21.5 mg/mL, 22 mg/mL, 22.5 mg/mL, 23 mg/mL, 23.5 mg/mL, 24 mg/mL, 24.5 mg/mL, 25 mg/mL, 25.5 mg/mL, 26 mg/mL, 26. 5 mg/mL, 27 mg/mL, 27.5 mg/mL, 28 mg/mL, 28.5 mg/mL, 29 mg/mL, or 29.5 mg/mL, while the sub-range high endpoint may be selected from 1.5 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 10.5 mg/mL, 11 mg/mL. 11.5 mg/mL, 12 mg/mL, 12.5 mg/mL, 13 mg/mL, 13.5 mg/mL, 14 mg/mL, 14.5 mg/mL, 15 mg/mL, 15.5 mg/mL, 16 mg/mL, 16.5 mg/mL, 17 mg/mL, 17.5 mg/mL, 18 mg/mL, 18.5 mg/mL, 19 mg/mL, 19.5 mg/mL, 20 mg/mL, 20.5 mg/mL, 21 mg/mL, 21.5 mg/mL, 22 mg/mL, 22.5 mg/mL, 23 mg/mL, 23.5 mg/mL, 24 mg/mL, 24.5 mg/mL, 25 mg/mL, 25.5 mg/mL, 26 mg/mL, 26. 5 mg/mL, 27 mg/mL, 27.5 mg/mL, 28 mg/mL, 28.5 mg/mL, 29 mg/mL, 29.5 mg/mL, or 30.0 mg/mL, where the high endpoint selected is higher than the low endpoint selected. In an embodiment, the solubility of clemizole HCl in the pH 4.5 citrate buffer-glycerol solution may be 5 mg/mL, 10 mg/mL, or 15 mg/mL. In an embodiment, the solubility of clemizole HCl in the 80% pH 4.5 citrate buffer - 20% glycerol solution may be 5 mg/mL, 10 mg/mL, or 15 mg/mL.

In an embodiment, no single clemizole HCl metabolite or related degradation product was greater or equal to 0.5% and the total of all clemizole HCl metabolites or related degradation products was less than or equal to 3.0%. In an embodiment, an increase in any single clemizole HCl metabolite or related degradation product over time during storage was less than or equal to 0.1% from the previous time point. Storage time points and conditions are as described herein.

In an embodiment, the pharmaceutical composition comprises a preservative. In an embodiment, the preservative comprises, consists essentially of, or consists of at least one of potassium sorbate, sodium methyl parahydroxybenzoate, sodium ethyl parahydroxybenzoate, methyl parahydroxybenzoate, or ethyl parahydroxybenzoate. In an embodiment, the preservative is potassium sorbate. In an embodiment, the preservative comprises, consists essentially of, or consists of at least one of sodium methyl parahydroxybenzoate or sodium ethyl parahydroxybenzoate. In an embodiment, the preservative comprises, consists essentially of, or consists of at least one of methyl parahydroxybenzoate or ethyl parahydroxybenzoate. In an embodiment, the preservative comprises methyl parahydroxybenzoate and ethyl parahydroxybenzoate.

In an embodiment, the preservative is present at 0.01-0.5% (w/v) in the pharmaceutical solution. In an embodiment, the preservative may be in 0.01% increments in the range of 0.01-0.5% (w/v) in the pharmaceutical solution. In an embodiment, the preservative may be selected from 0.01% (w/v), 0.02% (w/v), 0.03% (w/v), 0.04% (w/v), 0.05% (w/v), 0.06% (w/v), 0.07% (w/v), 0.08% (w/v), 0.09% (w/v), 0.1% (w/v), 0.11% (w/v), 0.12% (w/v), 0.13% (w/v), 0.14% (w/v), 0.15% (w/v), 0.16% (w/v), 0.17% (w/v), 0.18% (w/v), 0.19% (w/v), 0.2% (w/v), 0.21% (w/v), 0.22% (w/v), 0.23% (w/v), 0.24% (w/v), 0.25% (w/v), 0.26% (w/v), 0.27% (w/v), 0.28% (w/v), 0.29% (w/v), 0.30% (w/v), 0.31% (w/v), 0.32% (w/v), 0.33% (w/v), 0.34% (w/v), 0.35% (w/v), 0.36% (w/v), 0.37% (w/v), 0.38% (w/v), 0.39% (w/v), 0.4% (w/v), 0.41% (w/v), 0.42% (w/v), 0.43% (w/v), 0.44% (w/v), 0.45% (w/v), 0.46% (w/v), 0.47% (w/v), 0.48% (w/v), 0.49% (w/v), or 0.5% (w/v). In an embodiment, the preservative comprises, consists essentially of, or consists of at least one of sodium methyl parahydroxybenzoate, sodium ethyl parahydroxybenzoate, methyl parahydroxybenzoate, or ethyl parahydroxybenzoate. In an embodiment, the preservative comprises, consists essentially of, or consists of at least one of 0.2% (w/v) sodium methyl parahydroxybenzoate or 0.02% (w/v) sodium ethyl parahydroxybenzoate. In an embodiment, the preservative comprises, consists essentially of, or consists of at least one of 0.1% (w/v) sodium methyl parahydroxybenzoate or 0.01% (w/v) sodium ethyl parahydroxybenzoate. In an embodiment, the preservative comprises, consists essentially of, or consists of at least one of 0.2% (w/v) methyl parahydroxybenzoate or 0.02% (w/v) ethyl parahydroxybenzoate.

In an embodiment, the preservative comprises potassium sorbate. In an embodiment, the potassium sorbate is present in the range of 0.1% to 0.3% (w/v) in the pharmaceutical composition. In an embodiment, the potassium sorbate may be in 0.01% increments in the range of 0.1-0.3% (w/v) in the pharmaceutical solution. In an embodiment, the preservative may be selected from 0.1% (w/v), 0.11% (w/v), 0.12% (w/v), 0.13% (w/v), 0.14% (w/v), 0.15% (w/v), 0.16% (w/v), 0.17% (w/v), 0.18% (w/v), 0.19% (w/v), 0.2% (w/v), 0.21% (w/v), 0.22% (w/v), 0.23% (w/v), 0.24% (w/v), 0.25% (w/v), 0.26% (w/v), 0.27% (w/v), 0.28% (w/v), 0.29% (w/v), or 0.30% (w/v) in the pharmaceutical composition. In an embodiment, the potassium sorbate is present at 0.2% (w/v) in the pharmaceutical composition.

In an embodiment, the pharmaceutical composition comprises an antioxidant or a component with antioxidant properties. In an embodiment, the antioxidant or the component with antioxidant properties comprises, consists essentially of, or consists of at least one of sodium ascorbate, ascorbic acid, ethylenediaminetetraacetic acid (EDTA), or TPGS. In an embodiment, the antioxidant or the component with antioxidant properties is ascorbic acid. In an embodiment, the antioxidant or the component with antioxidant properties is ascorbic acid and EDTA. In an embodiment, the antioxidant or the component with antioxidant properties is sodium ascorbate. In an embodiment, the antioxidant or the component with antioxidant properties is sodium ascorbate and EDTA. In an embodiment, the antioxidant or the component with antioxidant properties is TPGS.

In an embodiment, the pharmaceutical composition comprising, consisting essentially of, or consisting of sodium methyl parahydroxybenzoate and sodium ethyl parahydroxybenzoate comprises a solubilizer. In an embodiment, the solubilizer may be TPGS, PEG400, or super-refined PEG400. In an embodiment, the solubilizer may be TPGS.

In an embodiment, the pharmaceutical composition comprising, consisting essentially of, or consisting of methyl parahydroxybenzoate and ethyl parahydroxybenzoate comprises a solubilizer. In an embodiment, the solubilizer may be TPGS, PEG400, super-refined PEG400, or propylene glycol.

In an embodiment, the preservative is stable for 1 day to 36 months. In an embodiment, the preservative is stable for 1 day, 1 week, 2 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, 36 months, or in a sub-range thereof between any of the time periods disclosed in this paragraph. The sub-range low endpoint may be selected from 1 day, 1 week, 2 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, or 35 months, while the sub-range high endpoint may be selected from 1 week, 2 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months, where the high endpoint selected is higher than the low endpoint selected. In an embodiment, the preservative is stable for 1 day to 1 week, 1 week to 2 weeks, 2 weeks to 1 month, 1 month to 2 months, 2 months to 3 months, 3 months to 6 months, 6 months to 9 months, 9 months to 12 months, 12 months to 18 months, 18 months to 24 months, 24 months to 36 months, or in a sub-range thereof. In an embodiment, the stability of the preservative may be such that greater than 95% of the preservative is stable. In an embodiment, the preservative is stable for at least one of 1 month, 2 months, 3 months, 6 months, 12 months, 18 months, 24 months, or 36 months.

In an embodiment, the preservative is stable for up to 36 months. In an embodiment, the preservative is stable for up to 1 day, 1 week, 2 weeks, 1 month, 2 months, 3 months, 4 months, 5months, 6 months, 7 months, 8 months, 9 months, 10 months, 11 months, 12 months, 13 months, 14 months, 15 months, 16 months, 17 months, 18 months, 19, months, 20 months, 21 months, 22 months, 23 months, 24 months, 25 months, 26 months, 27 months, 28 months, 29 months, 30 months, 31 months, 32 months, 33 months, 34 months, 35 months, 36 months, or in a sub-range thereof between any of the time periods disclosed in this paragraph. The sub-range low endpoint may be selected from 1 day, 1 week, 2 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, or 35 months, while the sub-range high endpoint may be selected from 1 week, 2 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months, where the high endpoint selected is higher than the low endpoint selected. The period of stability may be 1 day to 1 week, 1 week to 2 weeks, 2 weeks to 1 month, 1 month to 2 months, 2 months to 3 months, 3 months to 6 months, 6 months to 9 months, 9 months to 12 months, 12 months to 18 months, 18 months to 24 months, 24 months to 36 months, or in a sub-range thereof. In an embodiment, the stability of the preservative may be such that greater than 95% of the preservative is stable. In an embodiment, the preservative is stable for up to 1 month, 2 months, 3 months, 6 months, 12 months, 18 months, 24 months, or 36 months.

In an embodiment, the preservative is stable for at least 36 months. In an embodiment, the preservative is stable for at least 1 day, 1 week, 2 weeks, 1 month, 2 months, 3 months, 4 months, 5months, 6 months, 7 months, 8 months, 9 months, 10 months, 11 months, 12 months, 13 months, 14 months, 15 months, 16 months, 17 months, 18 months, 19, months, 20 months, 21 months, 22 months, 23 months, 24 months, 25 months, 26 months, 27 months, 28 months, 29 months, 30 months, 31 months, 32 months, 33 months, 34 months, 35 months, or 36 months. In an embodiment, the preservative is stable for any period of time in a range between any of the selected time periods described in this paragraph. The sub-range low endpoint may be selected from 1 day, 1 week, 2 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, or 35 months, while the sub-range high endpoint may be selected from 1 week, 2 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months, where the high endpoint selected is higher than the low endpoint selected. The period of stability may be 1 day to 1 week, 1 week to 2 weeks, 2 weeks to 1 month, 1 month to 2 months, 2 months to 3 months, 3 months to 6 months, 6 months to 9 months, 9 months to 12 months, 12 months to 18 months, 18 months to 24 months, 24 months to 36 months, or in a sub-range thereof. In an embodiment, the stability of the preservative may be such that greater than 95% of the preservative is stable. In an embodiment, the preservative is stable for at least one of 1 month, 2 months, 3 months, 6 months, 12 months, 18 months, 24 months, or 36 months.

In an embodiment, the stability of the preservative may be such that 90% to 100% is stable and free from degradation. In an embodiment, the stability of the preservative may be such that the preservative is stable in 0.5% increments in the range of 90% to 100% (that is, the preservative may be stable and present at 90%, 90.5%, 91%, 91.5%, 92%. 92.5 %, 93%, 93.5%, 94%, 94.5%, 95%, 95.5%, 96%, 96.5%, 97%, 97.5%, 98%, 98.5%, 99%, 99.5%, or 100%). The sub-range low endpoint may be selected from 90%, 90.5%, 91%, 91.5%, 92%. 92.5 %, 93%, 93.5%, 94%, 94.5%, 95%, 95.5%, 96%, 96.5%, 97%, 97.5%, 98%, 98.5%, 99%, 99.5%, while the sub-range high endpoint may be selected from 90.5%, 91%, 91.5%, 92%. 92.5 %, 93%, 93.5%, 94%, 94.5%, 95%, 95.5%, 96%, 96.5%, 97%, 97.5%, 98%, 98.5%, 99, 99.5%, or 100%, where the high endpoint selected is higher than the low endpoint selected. In an embodiment, the stability of the preservative is greater in the presence of an antioxidant.

In an embodiment, the stability of the preservative may be such that 90% to 100% is stable following storage for up until 0 months to 3 month, 3 months to 6 months, 6 months to 12 months, 12 months to 24 month, or 24 to 36 months of storage. In an embodiment, the stability of the preservative may be such that 90% to 95% or 95% to 100% is stable following up to 18 months of storage. In an embodiment, the stability of the preservative may be such that 90% to 95% or 95% to 100% is stable following up to 24 months of storage. In an embodiment, the stability of the preservative may be such that 90% to 95% or 95% to 100% is stable following up to 36 months of storage. In an embodiment, the stability of the preservative is greater in the presence of an antioxidant. In an embodiment, the preservative activity did not change by 5% or more over time during storage up to 0 months to 3 month, 3 months to 6 months, 6 months to 12 months, 12 months to 24 month, or 24 to 36 months from the preservative activity determined at the initial storage time point. Storage time points and conditions are as described herein.

In an embodiment, the preservative is stable at frozen temperatures, 0 °C, 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16°C, 17 °C, 18°C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, or 50 °. In an embodiment, the preservative is stable at any temperature in a range between any of the temperatures described in this paragraph. The sub-range low endpoint may be selected from frozen, 0°C, 1 °C, 2 °C, 3 °C, 4 °C, 5 °C, 6 °C, 7 °C, 8 °C, 9 °C, 10°C, 11 °C, 12 °C, 13 °C, 14 °C, 15 °C, 16 °C, 17 °C, 18 °C, 19 °C, 20°C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, or 49°C, while the sub-range high endpoint may be selected from 0°C, 1 °C, 2 °C, 3 °C, 4 °C, 5 °C, 6 °C, 7 °C, 8 °C, 9 °C, 10 °C, 11 °C, 12 °C, 13 °C, 14°C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, or 50°C, where the high endpoint selected is higher than the low endpoint selected. In an embodiment, the preservative is stable at 5°C, 25°C, and 40°C. In an embodiment, the preservative is stable at frozen temperature to 0°C, 0°C to 5°C, 5°C to 10°C, 10°C to 15°C, 15°C to 20°C, 20°C to 25°C, 25°C to 30°C, 30°C to 35°C, 35°C to 40°C, 40°C to 45°C, 45°C to 50°C or in a sub-range thereof.

In an embodiment, the pharmaceutical composition comprises a solubilizer. In an embodiment, the solubilizer comprises, consists essentially of, or consists of at least one of TPGS, super-refined PEG400, or propylene glycol. In an embodiment, the solubilizer comprises, consists essentially of, or consists of at least one of TPGS or super-refined PEG400. In an embodiment, the solubilizer comprises super-refined PEG400. In an embodiment, the solubilizer comprises TPGS.

In an embodiment, the pharmaceutical composition comprises a taste modifier and/or a sweetener. In an embodiment, the taste modifier comprises, consists essentially of, or consists of at least one of a flavoring, a sweeting agent, a liquid cherry flavor, a liquid orange flavor, a liquid strawberry flavor, a liquid vanilla flavor, a powdered cherry flavor, a powered orange flavor, a powered strawberry flavor, a powdered vanilla flavor. In an embodiment, the taste modifier comprises a powdered cherry flavor. In an embodiment, the sweetener comprises, consists essentially of, or consists of sucralose or sodium saccharin. In an embodiment, the sweetener comprises sucralose. In an embodiment, taste-testing was performed by a panel of taste-testers who evaluated the formulations that had been spiked with bitrex to formulations that were not spiked with bitrex. In an embodiment, there was no discernable difference between the sweeteners used or the flavoring agent in terms of palatability. In an embodiment, all of the formulations comprising a sweetener, a flavor agent, and glycerol have acceptable taste.

In an embodiment, the stability of the clemizole HCl may be such that greater than 90% of the clemizole HCl is stable following incubation of the clemizole HCl-solvent solution for 1 day, 1 week, 2 weeks, 1 month, 2 months, 3 months, 4 months, 5months, 6 months, 7 months, 8 months, 9 months, 10 months, 11 months, 12 months, 13 months, 14 months, 15 months, 16 months, 17 months, 18 months, 19, months, 20 months, 21 months, 22 months, 23 months, 24 months, 25 months, 26 months, 27 months, 28 months, 29 months, 30 months, 31 months, 32 months, 33 months, 34 months, 35 months, or 36 months. In an embodiment, the stability of the clemizole HCl may be such that greater than 90% of the clemizole HCl is stable following incubation of the clemizole HCl-solvent solution for any of the selected time periods described in this paragraph or any sub-range of time between any of the selected time periods described in this paragraph. In an embodiment, the stability of the clemizole HCl may be such that greater than 95% of the clemizole HCl is stable following incubation of the clemizole HCl-solvent solution for any of the selected time periods described in this paragraph or any sub-range of time between any of the selected time periods described in this paragraph. In an embodiment, the stability of the clemizole HCl may be such that greater than 90% of the clemizole HCl is stable following incubation of the clemizole HCl-solvent solution for 1 day to 1 week, 1 week to 2 weeks, 2 weeks to 1 month, 1 month to 2 months, 2 months to 3 months, 3 months to 6 months, 6 months to 9 months, 9 months to 12 months, 12 months to 18 months, 18 months to 24 months, 24 months to 36 months, or in a sub-range thereof. The sub-range low endpoint may be selected from 1 day, 1 week, 2 weeks, 1 month, 2 months, 3 months, 6 months, 9 months, 12 months, 18 months, or 24 months, while the sub-range high endpoint may be selected from 1 week, 2 weeks, 1 month, 2 months, 3 months, 6 months, 9 months, 12 months, 18 months, 24 months, or 36 months, where the high endpoint selected is higher than the low endpoint selected. In an embodiment, the stability of the clemizole HCl may be such that greater than 90% of the preservative is stable. In an embodiment, the clemizole HCl is stable for at least one of 1 month, 2 months, 3 months, 6 months, 12 months, 18 months, 24 months, or 36 months. In an embodiment, the stability of the clemizole HCl may be such that greater than 95% of the preservative is stable. following incubation of the clemizole HCl-solvent solution for 1 day to 1 week, 1 week to 2 weeks, 2 weeks to 1 month, 1 month to 2 months, 2 months to 3 months, 3 months to 6 months, 6 months to 9 months, 9 months to 12 months, 12 months to 18 months, 18 months to 24 months, 24 months to 36 months, or in a sub-range thereof. The sub-range low endpoint may be selected from 1 day, 1 week, 2 weeks, 1 month, 2 months, 3 months, 6 months, 9 months, 12 months, 18 months, or 24 months, while the sub-range high endpoint may be selected from 1 week, 2 weeks, 1 month, 2 months, 3 months, 6 months, 9 months, 12 months, 18 months, 24 months, or 36 months, where the high endpoint selected is higher than the low endpoint selected.

In an embodiment, the clemizole HCl is stable for up to 36 months. In an embodiment, the clemizole HCl is stable for up to 1 day, 1 week, 2 weeks, 1 month, 2 months, 3 months, 4 months, 5months, 6 months, 7 months, 8 months, 9 months, 10 months, 11 months, 12 months, 13 months, 14 months, 15 months, 16 months, 17 months, 18 months, 19, months, 20 months, 21 months, 22 months, 23 months, 24 months, 25 months, 26 months, 27 months, 28 months, 29 months, 30 months, 31 months, 32 months, 33 months, 34 months, 35 months, or 36 months. In an embodiment, the clemizole HCl is stable for any period of time in a range between any of the selected time periods described in this paragraph. The period of stability may be 1 day to 1 week, 1 week to 2 weeks, 2 weeks to 1 month, 1 month to 2 months, 2 months to 3 months, 3 months to 6 months, 6 months to 9 months, 9 months to 12 months, 12 months to 18 months, 18 months to 24 months, 24 months to 36 months, or in a sub-range thereof. The sub-range low endpoint may be selected from 1 day, 1 week, 2 weeks 1 month, 2 months, 3 months, 4 months, 5months, 6 months, 7 months, 8 months, 9 months, 10 months, 11 months, 12 months, 13 months, 14 months, 15 months, 16 months, 17 months, 18 months, 19, months, 20 months, 21 months, 22 months, 23 months, 24 months, 25 months, 26 months, 27 months, 28 months, 29 months, 30 months, 31 months, 32 months, 33 months, 34 months, or 35 months, while the sub-range high endpoint may be selected from 1 week, 2 weeks, 1 month, 2 months, 3 months, 4 months, 5months, 6 months, 7 months, 8 months, 9 months, 10 months, 11 months, 12 months, 13 months, 14 months, 15 months, 16 months, 17 months, 18 months, 19, months, 20 months, 21 months, 22 months, 23 months, 24 months, 25 months, 26 months, 27 months, 28 months, 29 months, 30 months, 31 months, 32 months, 33 months, 34 months, 35 months, or 36 months, where the high endpoint selected is higher than the low endpoint selected. In an embodiment, the stability of the clemizole HCl may be such that greater than 90% of the preservative is stable. In an embodiment, the stability of the clemizole HCl may be such that greater than 95% of the preservative is stable. In an embodiment, the clemizole HCl is stable for up to 1 month, 2 months, 3 months, 6 months, 12 months, 18 months, 24 months, or 36 months.

In an embodiment, the clemizole HCl is stable for at least 36 months. In an embodiment, the clemizole HCl is stable for at least 1 day, 1 week, 2 weeks, 1 month, 2 months, 3 months, 4 months, 5months, 6 months, 7 months, 8 months, 9 months, 10 months, 11 months, 12 months, 13 months, 14 months, 15 months, 16 months, 17 months, 18 months, 19, months, 20 months, 21 months, 22 months, 23 months, 24 months, 25 months, 26 months, 27 months, 28 months, 29 months, 30 months, 31 months, 32 months, 33 months, 34 months, 35 months, or 36 months. In an embodiment, the clemizole HCl is stable for any period of time in a range between any of the selected time periods described in this paragraph. The period of stability may be 1 day to 1 week, 1 week to 2 weeks, 2 weeks to 1 month, 1 month to 2 months, 2 months to 3 months, 3 months to 6 months, 6 months to 9 months, 9 months to 12 months, 12 months to 18 months, 18 months to 24 months, 24 months to 36 months, or in a sub-range thereof. The sub-range low endpoint may be selected from 1 day, 1 week, 2 weeks, 1 month, 2 months, 3 months, 4 months, 5months, 6 months, 7 months, 8 months, 9 months, 10 months, 11 months, 12 months, 13 months, 14 months, 15 months, 16 months, 17 months, 18 months, 19, months, 20 months, 21 months, 22 months, 23 months, 24 months, 25 months, 26 months, 27 months, 28 months, 29 months, 30 months, 31 months, 32 months, 33 months, 34 months, or 35 months, while the sub-range high endpoint may be selected from 1 week, 2 weeks, 1 month, 2 months, 3 months, 4 months, 5months, 6 months, 7 months, 8 months, 9 months, 10 months, 11 months, 12 months, 13 months, 14 months, 15 months, 16 months, 17 months, 18 months, 19, months, 20 months, 21 months, 22 months, 23 months, 24 months, 25 months, 26 months, 27 months, 28 months, 29 months, 30 months, 31 months, 32 months, 33 months, 34 months, 35 months, or 36 months, where the high endpoint selected is higher than the low endpoint selected. In an embodiment, the stability of the clemizole HCl may be such that greater than 90% of the preservative is stable. In an embodiment, the stability of the clemizole HCl may be such that greater than 95% of the preservative is stable. In an embodiment, the clemizole HCl is stable for at least one of 1 month, 2 months, 3 months, 6 months, 12 months, 18 months, 24 months, or 36 months.

In an embodiment, the stability of the clemizole HCl in the solvent may be such that 90% to 100% is stable and free from degradation. In an embodiment, the clemizole HCl may be stable from 0.5% increments in the range of 90% to 100% (that is, the clemizole HCl may be stable and present at 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, 99.5%, or 100%. The sub-range low endpoint may be selected from 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5%, while the sub-range high endpoint may be selected from 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, 99.5%, or 100%, where the high endpoint selected is higher than the low endpoint selected.

In an embodiment, the stability of the clemizole HCl in the solvent may be such that greater than 90% of the clemizole HCl is stable following incubation of the clemizole HCl-solvent solution at frozen temperature to 0°C, 0°C to 5°C, 5°C to 10°C, 10°C to 15°C, 15°C to 20°C, 20°C to 25°C, 25°C to 30°C, 30°C to 35°C, 35°C to 40°C, 40°C to 45°C, 45°C to 50°C or in a sub-range thereof. The sub-range low endpoint may be selected from frozen, 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31 °C, 32°C, 33°C, 34°C, 35°C, 36°C, 37°C, 38°C, 39°C, 40°C, 41°C, 42°C, 43°C, 44°C, 45 C, 46°C, 47°C, 48°C, or 49°C, while the sub-range high endpoint may be selected from 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31 °C, 32°C, 33°C, 34°C, 35°C, 36°C, 37°C, 38°C, 39°C, 40°C, 41 °C, 42°C, 43°C, 44°C, 45 C, 46°C, 47°C, 48°C, 49°C, or 50°C, where the high endpoint selected is higher than the low endpoint selected. In an embodiment, the stability of the clemizole HCl in the solvent may be such that greater than 95% of the clemizole HCl is stable following incubation of the clemizole HCl-solvent solution for any of the time periods described herein or any of the temperatures described in this paragraph.

In an embodiment, the stability of the clemizole HCl in the solvent may be such that greater than 90% of the clemizole HCl is stable following incubation of the clemizole HCl-solvent solution for up to 18 months at temperatures greater than 4°C but less than 40°C. In an embodiment, the stability of the clemizole HCl in the solvent may be such that greater than 90% of the clemizole HCl is stable following incubation of the clemizole HCl-solvent solution for up to 24 months at temperatures greater than 4°C but less than 40°C. In an embodiment, the stability of the clemizole HCl in the solvent may be such that greater than 90% of the clemizole HCl is stable following incubation of the clemizole HCl-solvent solution for up to 36 months at temperatures greater than 4°C but less than 40°C. In an embodiment, the stability of the clemizole HCl in the solvent may be such that greater than 90% of the clemizole HCl is stable following incubation of the clemizole HCl-solvent solution for up until 18 months at 25°C. In an embodiment, the stability of the clemizole HCl in the solvent may be such that greater than 90% of the clemizole HCl is stable following incubation of the clemizole HCl-solvent solution for up until 24 months at 25°C. In an embodiment, the stability of the clemizole HCl in the solvent may be such that greater than 90% of the clemizole HCl is stable following incubation of the clemizole HCl-solvent solution for up until 36 months at 25°C. In an embodiment, the stability of the clemizole HCl in the solvent may be such that greater than 90% of the clemizole HCl is stable following incubation of the clemizole HCl-solvent solution for up until 6 months at 40°C.

In an embodiment, the liquid pharmaceutical composition may comprise, consist essentially of, or consist of clemizole HCl, a pH 4.5 citrate buffer, a preservative, glycerol, a taste modifier, and a sweetener.

In an embodiment, the liquid pharmaceutical composition comprises of clemizole HCl, a pH 4.5 citrate buffer, glycerol, a taste modifier, a sweetener, super-refined PEG400, and the preservative is methyl parahydroxybenzoate and ethyl parahydroxybenzoate.

In an embodiment, the liquid pharmaceutical composition comprises of clemizole HCl, a pH 4.5 citrate buffer, glycerol, a taste modifier, a sweetener, TPGS, and the preservative is methyl parahydroxybenzoate and ethyl parahydroxybenzoate.

In an embodiment, the liquid pharmaceutical composition comprises of clemizole HCl, a pH 4.5 citrate buffer, glycerol, a taste modifier, a sweetener, TPGS, and the preservative is sodium methyl parahydroxybenzoate and sodium ethyl parahydroxybenzoate.

In an embodiment, the liquid pharmaceutical composition comprises of clemizole HCl, a pH 4.5 citrate buffer, glycerol, a taste modifier, a sweetener, TPGS, and the preservative is potassium ascorbate.

In an embodiment, the liquid pharmaceutical composition comprises of clemizole HCl, a pH 4.5 citrate buffer, glycerol, a taste modifier, a sweetener, sodium EDTA, ascorbic acid, and the preservative is potassium ascorbate.

In an embodiment, the liquid pharmaceutical composition further comprises an anti-epileptic drug.

In an embodiment, the liquid pharmaceutical composition may have a continuous flow and be readily passed through a syringe.

In an embodiment, the clemizole HCl is stable during storage. In an embodiment storage may be in the range of 2°C to 40°C. In an embodiment, storage may be as long as 6 months. In an embodiment, storage may be as long as 12 months. In an embodiment, storage may be as long as 18 months. In an embodiment, storage may be as long as 24 months. In an embodiment, storage may be as long as 36 months.

In an embodiment, the preservative is stable during storage. In an embodiment, storage temperature may be in the range of 2°C to 40°C. In an embodiment, the stability of the preservative may be such that greater than 90%, or greater than 95%, of the preservative is stable following incubation for 1 day to 1 week, 1 week to 2 weeks, 2 weeks to 1 month, 1 month to 2 months, 2 months to 3 months, 3 months to 6 months, 6 months to 9 months, 9 months to 12 months, 12 months to 18 months, 18 months to 24 months, 24 months to 36 months, or in a sub-range thereof. The sub-range low endpoint may be selected from 1 day, 1 week, 2 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, or 35 months, while the sub-range high endpoint may be selected from 1 week, 2 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months, where the high endpoint selected is higher than the low endpoint selected. In an embodiment, the stability of the preservative may be such that greater than 90% of the preservative is stable following incubation for at least one of 1 day, 1 week, 1 month, 2 months, 3 months, 6 months, 12 months, 18 months, 24 months, or 36 months. In an embodiment, the stability of the preservative may be such that greater than 95% of the preservative is stable following incubation for at least one of 1 day, 1 week, 1 month, 2 months, 3 months, 6 months, 12 months, 18 months, 24 months, or 36 months.

In an embodiment, the stability of the preservative in the solution may be such that greater than 90% of the preservative is stable following incubation of the solution at frozen temperature to 0°C, 0°C to 5°C, 5°C to 10°C, 10°C to 15°C, 15°C to 20°C, 20°C to 25°C, 25°C to 30°C, 30°C to 35°C, 35°C to 40°C, 40°C to 45°C, 45°C to 50°C, or in a sub-range thereof. The sub-range low endpoint may be selected from frozen, 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41 °C, 42°C, 43°C, 44°C, 45 C, 46°C, 47°C, 48°C, or 49°C, while the sub-range high endpoint may be selected from 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31 °C, 32°C, 33°C, 34°C, 35°C, 36°C, 37°C, 38°C, 39°C, 40°C, 41°C, 42°C, 43°C, 44°C, 45 C, 46°C, 47°C, 48°C, or 49°C, while the sub-range high endpoint may be selected from 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31 °C, 32°C, 33°C, 34°C, 35°C, 36°C, 37°C, 38°C, 39°C, 40°C, 41°C, 42°C, 43°C, 44°C, 45 C, 46°C, 47°C, 48°C, or 49°C, while the sub-range high endpoint may be selected from 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45 C, 46°C, 47°C, 48°C, 49°C, or 50°C, where the high endpoint selected is higher than the low endpoint selected. In an embodiment, the stability of the preservative in the solvent may be such that greater than 95% of the preservative is stable following incubation of the solution for any of the time periods or temperatures described in this paragraph. In an embodiment, the stability of the preservative in the solvent may be such that greater than 90% of the preservative is stable following incubation of the solution at 2°C to 8°C, 25°C, or 40°C. In an embodiment, the stability of the preservative in the solvent may be such that greater than 95% of the preservative is stable following incubation of the solution at 2°C to 8°C, 25°C, or 40°C. In an embodiment, the stability of the preservative in the solvent may be such that greater than 95% of the preservative is stable following incubation of the solution at 2°C to 8°C, 25°C, or 40°C for up to 1 day, 1 week, 1 month, 2 months, 3 months, 6 months, 12 months, 24 months, or 36 months, or any time sub-range of time in between these times.

In an embodiment, the liquid pharmaceutical formulation is in the form of an oral formulation. In an embodiment, the oral formulation may further comprise at least one of a pharmaceutically acceptable carrier. In an embodiment, the pharmaceutically acceptable carrier may include any one or more agents selected from the group consisting of ion exchangers, alumina, aluminum stearate, lecithin, serum proteins, human serum albumin, buffer substances, phosphates, glycine, sorbic acid, potassium sorbate, partial glyceride mixtures of saturated vegetable fatty acids, water, salts, electrolytes, protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, waxes, polyethylene glycol, starch, lactose, dicalcium phosphate, microcrystalline cellulose, sucrose, talc, magnesium carbonate, kaolin, non-ionic surfactants, edible oils, physiological saline, bacteriostatic water, polyethoxylated castor oil, phosphate buffered saline (PBS), complexation agents including but not limited to cyclodextrins, or any other suitable pharmaceutically acceptable carrier

In an embodiment, the liquid pharmaceutical formulation comprises a further pharmaceutically acceptable excipient. In an embodiment, the pharmaceutically acceptable excipient may include any one or more substances selected from the group consisting of an acidifying agent, an alkalizing agent, an antiadherent, an anticaking agent, an antifoaming agent, an antimicrobial a preservative, an antioxidant, a binder, a coating agent, a color, a disintegrant, a diluent, an emulsifying agent, an extended release agent, a flavor, a glidant, a humectant, a lubricant, an ointment base, a preservative, a solubilizer, a sorbent, a sustained release agent, a sweetening agent, or any other suitable pharmaceutically acceptable excipient.

In an embodiment, the method of making the liquid pharmaceutical formulation comprises, consists essentially of, or consists of dissolving at least one preservative in a solution comprising at least one of a solvent, a solubilizer, or a combined solvent-solubilizer solution; dissolving clemizole HCl in a solution comprising at least one of the solvent, the solubilizer, or the combined solvent-solubilizer solution; dissolving a plurality of excipients in a solution comprising at least one of a solvent, a solubilizer, or a combined solvent-solubilizer solution;_adding glycerol; and adjusting the pH. In an embodiment, the solvent comprises, consists essentially of, or consists of a citrate buffer having pH 4.5 +/-0.1. In an embodiment, the pH of the liquid pharmaceutical composition is 4.5 (+/- 0.5). In an embodiment, the final concentration of clemizole HCl in the liquid pharmaceutical composition ranges from 1 mg/mL - 30 mg/mL. In an embodiment, the at least one preservative comprises, consists essentially of, or consists of at least one of potassium sorbate, sodium methyl parahydroxybenzoate, sodium ethyl parahydroxybenzoate, methyl parahydroxybenzoate, or ethyl parahydroxybenzoate.

In an embodiment, the method of making the liquid pharmaceutical formulation comprises dissolving the at least one preservative in a solubilizer; adding solvent to the solubilizer-preservative solution; dissolving the clemizole HCl in the solubilizer-preservative-solvent solution, dissolving the plurality of excipients in the clemizole HCl-solubilizer-preservative-solvent solution; adding glycerol to the resulting solution; and adjusting the pH to 4.5 (+/- 0.5) to form the liquid pharmaceutical composition. The preservative is at least one of methyl parahydroxybenzoate or ethyl parahydroxybenzoate, the solvent is citrate buffer having pH 4.5 +/-0.1, and the solubilizer is super-refined PEG400. In an embodiment, the TPGS is melted and kept at approximately 50°C. In an embodiment, the plurality of excipients comprises at least one sweetener and at least one taste modifier.

In an embodiment, the method of making the liquid pharmaceutical formulation comprises adding the solubilizer to the solvent; dissolving the clemizole HCl in the combined solvent-solubilizer solution; dissolving the at least one preservative in the clemizole HCl-solvent-solubilizer solution; dissolving the plurality of excipients in the preservative-clemizole HCl-solvent-solubilizer solution; adding glycerol to the resulting solution; and adjusting the pH to 4.5 (+/- 0.5) to form the liquid pharmaceutical composition. The preservative is at least one of sodium methyl parahydroxybenzoate or sodium ethyl parahydroxybenzoate, and the solvent is citrate buffer having pH 4.5 +/-0.1. In an embodiment, the solubilizer is super-refined PEG400. In an embodiment, the solubilizer is TPGS, and the TPGS is melted prior to combining it with the solvent. In an embodiment, the plurality of excipients comprises at least one sweetener and at least one taste modifier.

In an embodiment, the method of making the liquid pharmaceutical formulation comprises adding the solubilizer to the solvent; dissolving the at least one preservative in the combined solvent-solubilizer solution; dissolving the plurality of excipients in the preservative-solvent-solubilizer solution; adding the glycerol to the excipient-preservative-solvent-solubilizer solution; dissolving the clemizole HCl in the excipient-preservative-solvent-solubilizer solution; and adjusting the pH to 4.5 (+/-0.5) to form the liquid pharmaceutical composition. The preservative comprises potassium sorbate, the solvent is citrate buffer having pH 4.5 +/-0.1, and the solubilizer is TPGS. In an embodiment, the TPGS is melted prior to combining it with the solvent. In an embodiment, the plurality of excipients comprises at least one sweetener and at least one taste modifier.

In an embodiment, the method of making the liquid pharmaceutical formulation comprises dissolving the at least one preservative in the solvent; dissolving an anti-oxidant in the preservative-solvent solution; dissolving the plurality of excipients in the anti-oxidant-preservative-solvent solution; adding the glycerol to the excipient-anti-oxidant-preservative-solvent solution; dissolving the clemizole HCl in the excipient-anti-oxidant-preservative-solvent solution; and adjusting the pH to 4.5 (+/- 0.5) to form the liquid pharmaceutical composition. The solvent is citrate buffer having pH 4.5 +/-0.1, and the anti-oxidant comprises, consists essentially of, or consists of sodium EDTA, sodium ascorbate, or ascorbic acid. In an embodiment, the anti-oxidant comprises, consists essentially of, or consists of sodium EDTA and ascorbic acid. In an embodiment, the plurality of excipients comprises at least one sweetener and at least one taste modifier.

In an embodiment, the method of making the liquid pharmaceutical formulation comprises adding an anti-epilepsy drug.

In an embodiment, a method of treating a subject having an epilepsy disorder comprises administering the liquid pharmaceutical composition of any of the embodiments described herein. In an embodiment, one or more routes selected from the group consisting of oral, sublingual, sublabial, buccal, and transmucosal may be used to administer the liquid pharmaceutical composition of any of the embodiments described herein. In an embodiment, the liquid pharmaceutical formulation is administered orally. In an embodiment, the epilepsy disorder is a pediatric epilepsy disorder.

In an embodiment, the concentration of clemizole HCl in the liquid pharmaceutical composition may be in the range of 1 mg/mL - 30 mg/mL.

In an embodiment, the dose of clemizole HCl used to treat the subject may be selected from 0.5 mg/kg to 500 mg/kg. In an embodiment, the dose of clemizole HCl used to treat the subject may be 0.5 mg/kg to 50 mg/kg, 50 mg/kg to 100 mg/kg, 100 mg/kg to 150 mg/kg, 150 mg/kg to 200 mg/kg, 200 mg/kg to 250 mg/kg, 250 mg/kg to 300 mg/kg, 300 mg/kg to 350 mg/kg, 350 mg/kg to 400 mg/kg, 400 mg/kg to 450 mg/kg, 450 mg/kg to 500 mg/kg, or any sub-range in between any of these dosing ranges. In an embodiment, the clemizole HCl used to treat the subject may be from 0.5 mg/kg increments in the range of 0.5 mg/kg to 500 mg/kg (that is, the dose used to treat a subject may be 0.5 mg/kg, 1 mg/kg, 1.5 mg/kg, 2 mg/kg, 2.5 mg/kg, 3 mg/kg...101 mg/kg, 101.5 mg/kg, 102 mg/kg... 498 mg/kg, 498.5 mg/kg, 499 mg/kg, 499.5 mg/kg, or 500 mg/kg). In an embodiment, the clemizole HCl used to treat the subject may be from 0.5 mg/kg to 500 mg/kg, or in a sub-range thereof. The sub-range low endpoint may be selected from 0.5 mg/kg, 1 mg/kg, 1.5 mg/kg, 2 mg/kg, 2.5 mg/kg, 3 mg/kg...100 mg/kg, 100.5 mg/kg, 101 mg/kg, 101.5 mg/kg, 102 mg/kg . . . 498 mg/kg, 498.5 mg/kg, 499 mg/kg, or 499.5 mg/kg, while the sub-range high endpoint may be selected from 1 mg/kg, 1.5 mg/kg, 2 mg/kg, 2.5 mg/kg, 3 mg/kg, 3.5 mg/kg, 4 mg/kg...101 mg/kg, 101.5 mg/kg, 102 mg/kg, 102.5 mg/kg, 103 mg/kg ... 498 mg/kg, 498.5 mg/kg, 499 mg/kg, 499.5 mg/kg, or 500 mg/kg, where the high endpoint selected is higher than the low endpoint selected. In an embodiment, the dose of clemizole HCl used to treat the subject may be 0.5 mg/kg, 1 mg/kg, 2.5 mg/kg, 5 mg/kg, 7.5 mg/kg, 10 mg/kg, 12.5 mg/kg, 15 mg/kg, 17.5 mg/kg, 20 mg/kg, 22.5 mg/kg, 25 mg/kg, 27.5 mg/kg, 30 mg/kg, 40 mg/kg, 42.5 mg/kg, 45 mg/kg, 47.5 mg/kg, 50 mg/kg, 52.5 mg/kg, 55 mg/kg, 57.5 mg/kg, 60 mg/kg, 62.5 mg/kg, 65 mg/kg, 67.5 mg/kg, 70 mg/kg, 72.5 mg/kg, 75 mg/kg, 77.5 mg/kg, 80 mg/kg, 82.5 mg/kg, 85 mg/kg, 87.5 mg/kg, 90 mg/kg, 92.5 mg/kg, 95 mg/kg, 97.5 mg/kg, 100 mg/kg, 125 mg/kg, 150 mg/kg, 175 mg/kg, 200 mg/kg, 225 mg/kg, 250 mg/kg, 275 mg/kg, 300 mg/kg, 325 mg/kg, 350 mg/kg, 375 mg/kg, 400 mg/kg, 425 mg/kg, 450 mg/kg, 475 mg/kg or 500 mg/kg. In an embodiment, the dose of clemizole HCl used to treat the subject may be 50 mg/kg, 100 mg/kg, 200 mg/kg, 300 mg/kg, 400 mg/kg, or 500 mg/kg. In an embodiment, the subject is a subject having a pediatric epilepsy disorder.

In an embodiment, the method of treating the subject comprises further treating the subject with an anti-epilepsy drug. In an embodiment, the anti-epilepsy drug is administered separately from the liquid pharmaceutical composition comprising clemizole HCl described herein. In an embodiment, the anti-epilepsy drug is administered simultaneously with the liquid pharmaceutical composition comprising clemizole HCl described herein. In an embodiment, the anti-epilepsy drug is administered sequentially with the liquid pharmaceutical composition comprising clemizole HCl described herein. In an embodiment, the subject is a subject having a pediatric epilepsy disorder.

In an embodiment, a method of treating the subject comprises administering the liquid pharmaceutical composition comprising clemizole HCl described herein at least once daily. In an embodiment, a method of treating the subject comprises administering the liquid pharmaceutical composition comprising clemizole HCl described herein 1 to 4 times daily. In an embodiment, a method of treating the subject comprises administering the liquid pharmaceutical composition comprising clemizole HCl described herein 1 to 3 times daily. In an embodiment, a method of treating the subject comprises administering the liquid pharmaceutical composition comprising clemizole HCl described herein 1 to 2 times daily, 2 to 3 times daily or 3 to 4 times daily. In an embodiment, a method of treating the subject comprises administering the liquid pharmaceutical composition comprising clemizole HCl described herein once daily, 2 times daily, 3 times daily, or 4 times daily. In an embodiment, a method of treating the subject comprises administering the liquid pharmaceutical composition comprising clemizole HCl described herein every second day. In an embodiment, a method of treating the subject comprises administering the liquid pharmaceutical composition at least every 4, 5, 6, 7, 8, 9, 10, 12, 16, 20, or 24 hours. In an embodiment, a method of treating the subject comprises administering the liquid pharmaceutical composition comprising clemizole HCl described herein as needed.

### EXAMPLES:

### Example 1:

Solubility testing for clemizole HCl in a compatible solvent system was conducted to identify a buffer system that solubilized the required concentration of clemizole HCl without any precipitation. A target concentration of clemizole HCl of 5 mg/mL was achieved in several solvents tested as indicated in the shaded rows of Table 1 below.

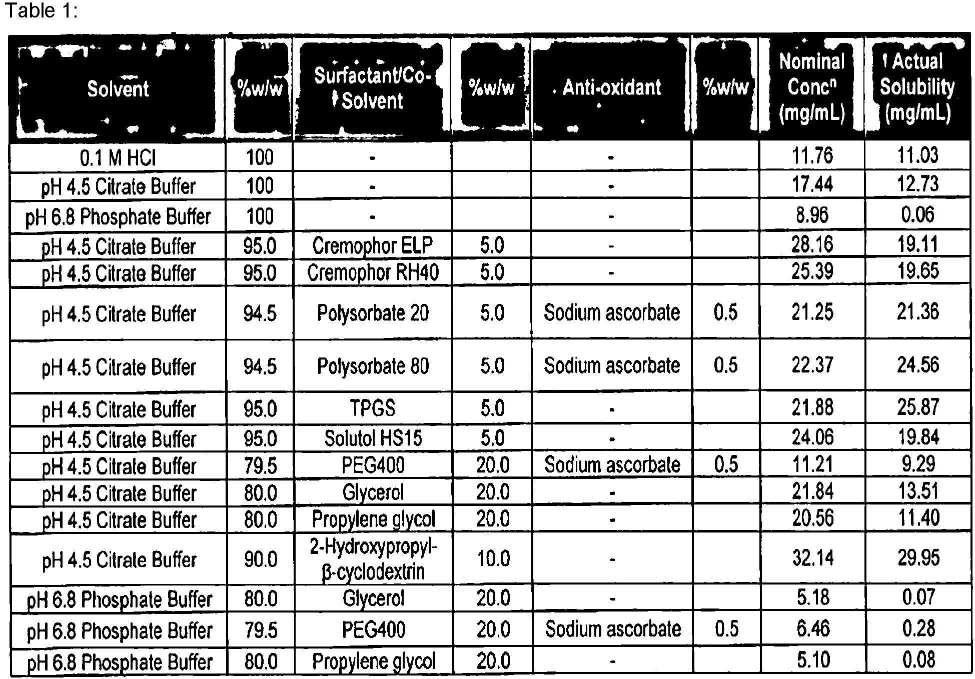

Clemizole HCl achieved target solubility in 0.1 M HCl; pH 4.5 citrate buffer; pH 4.5 citrate buffer-Cremophor ELP; pH 4.5 citrate buffer-Cremophor RH40; pH 4.5 citrate buffer-polysorbate 20; pH 4.5 citrate buffer-polysorbate 80; pH 4.5 citrate buffer-TPGS; pH 4.5 citrate buffer, pH 4.5 citrate buffer-solutol HS15; pH 4.5 citrate buffer-PEG400; pH 4.5 citrate buffer-glycerol; pH 4.5 citrate buffer-propylene glycol; and pH 4.5 citrate buffer-2-(hydroxypropyl-beta-cyclodextrin). The data indicate that clemizole HCl achieves target solubility of at least 5 mg/mL in several solvent solution combinations. Actual clemizole HCl solubility values in each of the solvent systems are described in Table 1.

Based on the observations from the data in Table 1, in was determined that clemizole HCl has a pH solubility profile being more soluble in acidic pH (0.1 M HCl and pH 4.5 citrate buffer) than in pH 6.8 phosphate buffer where it was almost completely insoluble. Addition of a cosolvent to pH 6.8 phosphate buffer did not improve the solubility at this pH as illustrated in Table 1. Table 1 also demonstrates that the addition of some cosolvents (glycerol, PEG400, and propylene glycol) although not synergistically, do increase the solubility of clemizole HCl in the pH 4.5 citrate buffer, whereas the addition of other cosolvents (Cremophor ELP, Cremophor RH40, Solutol HS15, Polysorbate 20 and sodium ascorbate, Polysorbate 80 and sodium ascorbate, TPGS, and 2-hydroxypropyl-betacyclodextrin) further improve the solubility of clemizole HCl in the pH 4.5 citrate buffer. Additional solvents/cosolvents tested included sodium lauryl sulphate (10% w/w in water) and sodium docusate

### (10% w/w in water).

Although not shown in Table 1 above, the solubility of clemizole HCl was enhanced to 36 mg/mL in a 20 %w/w solution of Solutol HS15 at 40°C. Also, freeze-thawing does not appear to reduce the clemizole HCl solubility suggesting that very little of the clemizole HCl precipitates upon cooling.

### Example 2:

In an example, based on the results from Example 1, pH 4.5 citrate buffer was used based on high solubility of clemizole HCl. Glycerol was added to aid in the taste-masking and to modify the viscosity. The viscosity of the glycerol/citrate buffer was assessed using a "syringeability" test to confirm the ease of dispensing before addition of clemizole HCl. Table 2 below describes the formulation of select concentrations of glycerol in pH 4.5 citrate buffer.

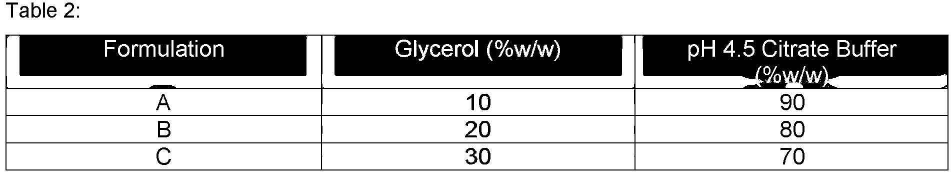

"Syringeability" of these formulations through two types of oral dosing syringes (Adelphi "Elm Tip," code 706100 and Adelphi Flat Tip, code 90500) were assessed following the addition of viscous glycerol. The syringeability data are summarized in Table 3, where the following coding applies: 1=Not possible to syringe (no flow); 2=Possible to syringe (drop-wise flow); 3=Ease of syringeability (moderate or continuous flow); 4=Easy to syringe (continuous flow).

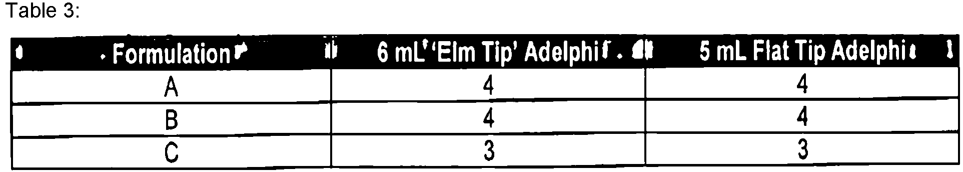

### Example 3:

In an example, clemizole HCl was added to each to each formulation from Table 2 in increasing amounts to assess the solubility of the clemizole HCl in the solvent mixture and to confirm that a solubility of 5 mg/mL clemizole HCl could be achieved. When 20 mg of clemizole HCl was added to each of the solvent mixtures from Table 2 (A1, B1, and C1) to achieve target concentrations of 5 mg/mL Clemizole HCl, full dissolution was achieved at approximately 4 minutes using a vortex mixer. When 30 mg of clemizole HCl was added to each of the solvent mixtures from Table 2 (A2, B2, and C2) to achieve target concentrations of 7.5 mg/mL clemizole HCl, full dissolution was achieved at approximately 4 minutes using a vortex mixer. When 40 mg of clemizole HCl was added to each of the solvent mixtures from Table 2 (A3, B3, and C3) to achieve target concentrations of 10 mg/mL Clemizole HCl, the samples were not fully dissolved at approximately 6 minutes of vortexing. The samples were then placed in an incubator at 25°C overnight after which the clemizole HCl was fully dissolved. All samples were analyzed for clemizole HCl content following overnight incubation at 25°C. Table 4 describes the measured solubility of clemizole HCl in each of the samples.

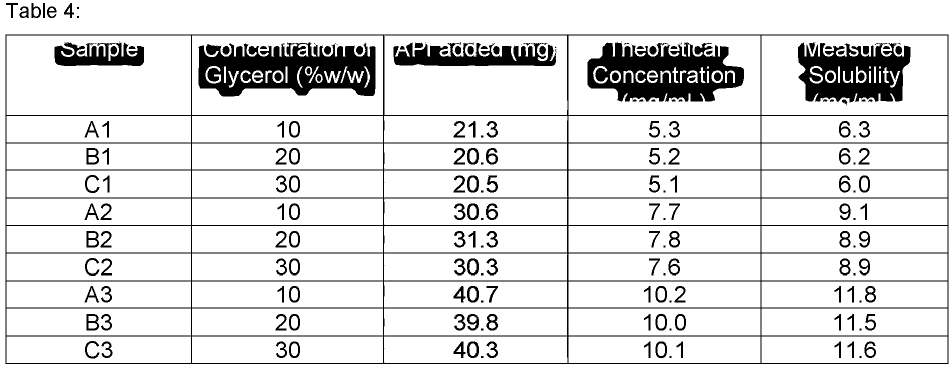

The solubility values were all slightly higher than expected, which may be due to inaccuracies in measuring the small volumes for the study. There were no related substances reported greater than 0.05% for any of the samples. The results confirm that it is possible to dissolve clemizole HCl at a concentration greater than 5 mg/mL containing 20% w/v glycerol because at this concentration of glycerol, the formulation would be readily syringeable.

### Example 4:

In an example, various excipients were examined at the 14-day time point (50°C) to guide the decision on the choice of excipients to be used during formulation development. Table 5 below illustrates the stability of clemizole HCl at day 14 following incubation in the various formulations listed.

**Table 5:**

| Composition (Excipient +API) | Assay Results (%) | | | Total Related Substances (Area %) | | |
|---|---|---|---|---|---|---|
| | Initial | Initial Mean | 50 °C, 14 day | 50 °C, 14 day Mean | Initial | 50 °C, 14 day |
| | 97.6 | | 91.4 | | | |
| pH 4.5 Acetate Buffer | 98.1 | 97.9 | 83.4 | 87.4 | - | - |
| pH 4.5 Citrate Buffer | 96.7 | 96.6 | 101.0 | 101.0 | - | - |
| | 96.5 | | 101.0 | | | |
| pH 4.5 Citrate Buffer/Cremophor ELP | 97.3 | 96.9 | 88.0 | 90.5 | 1.23 | 2.64 |
| | 96.5 | | 92.9 | | | |
| pH 4.5 Citrate Buffer/TPGS | 96.2 | 95.6 | 98.6 | 96.4 | - | 0.84 |
| | 95.0 | | 94.2 | | | |
| pH 4.5 Citrate Buffer/Polysorbate 80 | 94.8 | 95.0 | 99.9 | 92.6 | 2.50 | 3.67 |
| | 95.2 | | 85.3 | | | |
| pH 4.5 Citrate Buffer/Polysorbate 80/Sodium ascorbate | 95.1 | 95.6 | 91.8 | 89.6 | 0.07 | 1.77 |
| | 96.2 | | 87.4 | | | |
| pH 4.5 Citrate Buffer/PEG400 | 97.1 | 96.5 | 92.7 | 91.2 | - | 3.25 |
| | 95.9 | | 89.6 | | | |
| pH 4.5 Citrate Buffer/PEG400/sodium ascorbate | 94.9 | 95.4 | 94.3 | 94.0 | 0.11 | 0.75 |
| | 96.0 | | 93.8 | | | |
| pH 4.5 Citrate Buffer/cherry black powder | 97.2 | 97.5 | 101.4 | 101.3 | - | - |
| | 97.9 | | 101.3 | | | |
| pH 4.5 Citrate Buffer/cherry black liquid | 98.3 | 96.7 | 97.0 | 96.9 | - | - |
| | 95.2 | | 96.7 | | | |
| pH 4.5 Citrate Buffer/sucralose | 95.8 | 96.1 | 97.9 | 97.3 | - | 0.05 |
| | 96.5 | | 96.6 | | | |
| pH 4.5 Citrate Buffer/sodium Saccharin | 96.4 | 96.0 | 96.7 | 96.3 | - | - |
| | 95.6 | | 95.8 | | | |
| pH 4.5 Citrate Buffer/sodium methylparahydroxybenzoate/ethyl parahydroxybenzoate | 95.9 | 96.0 | 96.6 | 96.0 | 0.05 | 0.18 |
| | 96.2 | | 95.5 | | | |
| pH 4.5 Citrate Buffer/potassium sorbate | 97.1 | 99.3 | 94.6 | 95.1 | - | 0.08 |
| | 101.6 | | 95.5 | | | |
| Glycerol | 115.0 | 103.6 | 97.3 | 97.7 | - | 0.25 |
| | 92.2 | | 98.0 | | | |
| Prototype W* | 97.4 | 97.1 | 93.8 | 93.9 | - | 0.53 |
| | 96.7 | | 93.9 | | | |
| Prototype X* | 98.8 | 98.9 | 96.2 | 95.8 | - | 0.66 |
| | 98.9 | | 95.3 | | | |
| Prototype Y* | 96.5 | 97.1 | 93.8 | 94.3 | - | 0.77 |
| | 97.7 | | 94.7 | | | |
| Prototype Z* | 97.3 | 97.5 | 94.7 | 93.4 | 0.18 | 0.62 |
| | 97.5 | | 92.0 | | | |
| API only | 100.0 | 100.5 | 101.1 | 100.9 | - | - |
| | 101.0 | | 100.7 | | | |

Prototypes W, X, Y, and Z can be found in Table 6 below.

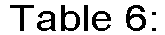

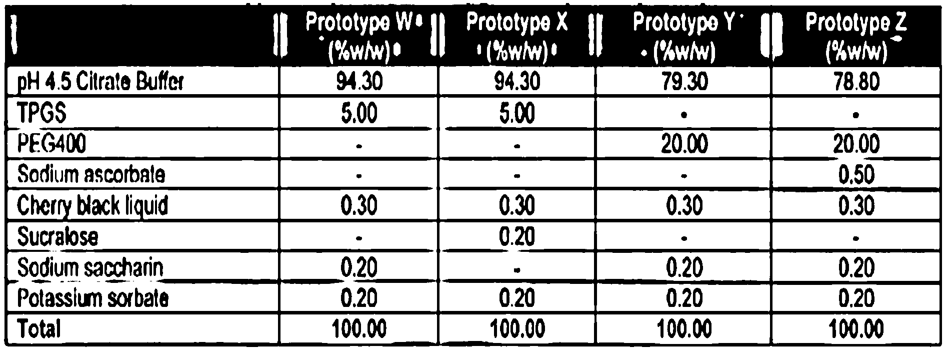

Based on the observations from Table 5, it was determined that pH 4.5 citrate buffer was compatible with clemizole HCl and target concentrations of at least 5 mg/mL clemizole HCl could be achieved; some of the solubilizers caused stability issues (although this may have been due to oxidation of the clemizole HCl due to the presence of peroxides in the excipients); two excipients that typically have low levels of peroxide (PEG400 and polysorbate 80) showed greater levels of related substances, which reduced when an anti-oxidant was included suggesting that clemizole HCl may be susceptible to oxidation; the sweeteners and flavorings tested were compatible and could be used for taste masking; and the preservatives, sodium methyl parahydroxybenzoate, sodium ethyl parahydroxybenzoate, and potassium sorbate showed little related substances. However, due to the poor solubility of ethyl parahydroxybenzoate, either a solubilizer such as PEG400, or a soluble form of ethyl parahydroxybenzoate, such as the sodium salt, would be required. Glycerol was compatible with clemizole HCl and may be used to improve the taste formulation.

### Example 5:

In an example, placebo formulations were prepared for taste testing. The samples were spiked with bitrex (denatonium benzoate) at 25 ppb w/v as a surrogate to simulate the bitterness of the clemizole HCl. Five placebo formulations were prepared according to Table 7. Placebo was also prepared based on knowledge that glycerol is known to interact with paraben preservatives over time, which were present in the samples as sodium methyl parahydroxybenzoate and sodium ethyl parahydroxybenzoate. Therefore, ideally it is preferred to avoid glycerol or polyols in general if parabens are used as preservative. However, if glycerol should be required for taste, then the decrease in preservative content did not preclude its use.

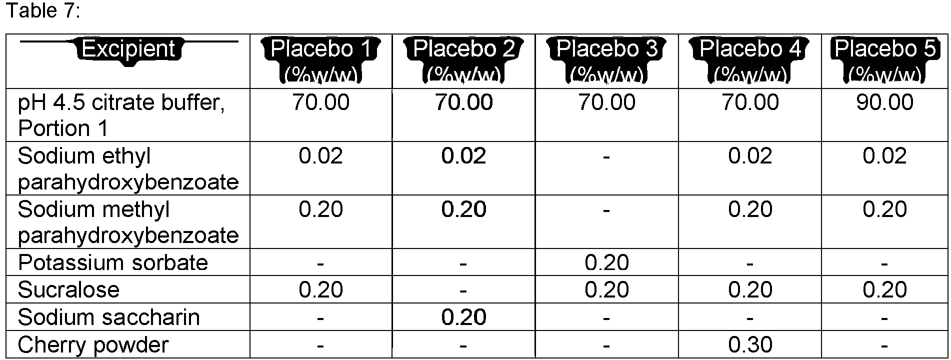

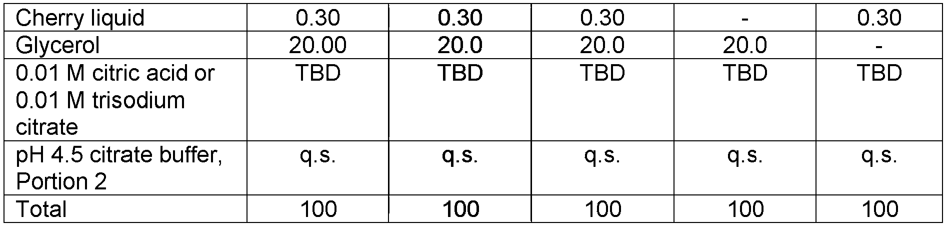

In the placebo formulations above, on addition of cherry liquid, the solution became amber in color and there were small globules present, which were likely due to the triacetin in the flavor formulation. These globules appeared to disperse into the bulk liquid on standing overnight. On addition of cherry powder as a flavor agent, the cherry powder dissolved to yield a colorless solution. All formulations apart from Placebo 3 required pH adjustment using citric acid because the pH prior to adjustment was between 5 and 7. A panel of four taste-testers evaluated the formulation, comparing the 5 Placebo formulations that had been spiked with bitrex to the 5 Placebo formulations that were not spiked with bitrex. The results from taste testing are represented in Table 8:

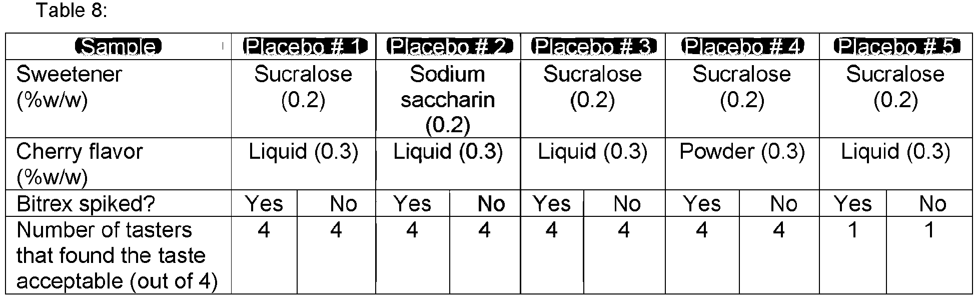

The results from the taste evaluation confirm the need for glycerol in the formulation based on at least the fact that only 1 of 4 of the taste-testers found the taste of the Placebo 5 formulation acceptable. In terms of choice of sweetener, there was no discernable difference between Placebo 1 (sucralose) and Placebo 2 (sodium saccharin). In terms of choice of cherry flavor, there was no discernable difference between Placebo 3 (cherry liquid) and Placebo 4 (cherry powder). Because powder cherry had a longer shelf life than liquid cherry, and its solubility was better in formulations than the liquid form, powder cherry was selected as the flavoring in examples. Because sucralose was previously used as a sweetener in other pediatric formulations, it was used as the sweetener in examples.

### Example 6:

In an example, the lead formulations from Example 4, including Table 7 and Table 8, were used to manufacture at a larger scale and to put on a short-term accelerated stability study. Modifications to each of these formulations were made, including addition of an anti-oxidant based on the potential requirement for one from the excipient compatibility studies given that clemizole HCl appeared to undergo some oxidation when in the presence of excipients that comprise peroxides (i.e. PEG400 and Tween 80); and use of an alternative preservative due to slightly low assay results obtained for both preservatives after 14 days at (50°C) as illustrated in Table 5. These assay results were not consistent with the very small amount of related substances observed for these samples and hence both preservatives remained options for progression. Based on the data obtained in the excipient compatibility study of Example 4, prototype batches were manufactured according to Table 9 below, which included two options for a preservative: Prototype A having potassium sorbate as a preservative, and Prototype B having sodium methyl parahydroxybenzoate and sodium ethyl parahydroxybenzoate as preservative. The prototype batches further distinguished between the presence of an antioxidant (Prototype AO, Prototype BO, as well as both placebos) and the absence of an antioxidant (Prototype A and Prototype B).

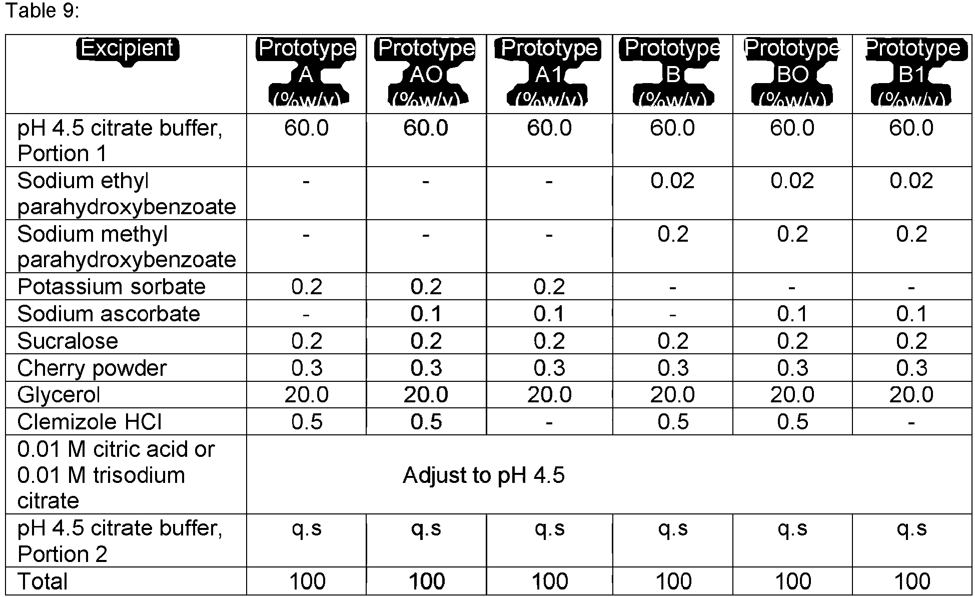

Prototype B and Prototype BO were not progressed due to a precipitate forming from an interaction between the parabens (sodium methyl parahydroxybenzoate and sodium ethyl parahydroxybenzoate) and clemizole HCl. Prototypes A, AO, and the placebo were placed onto the stability studies.

### Example 7:

In an example, stability studies were performed on Prototypes A, AO, and A1 over 6 months at various temperatures. Samples were tested for appearance, pH, specific gravity, assay (clemizole HCl), related substances, and preservative assay. Table 10 below summarizes the results of the appearance of the prototypes stored for 2 weeks, 1 month, or 2 months as indicated, and at temperatures of 2- 8°C, 25°C, or 40°C. These results demonstrate that the appearance of the formulations remained clear and free from particulates over the temperatures and time of incubation.

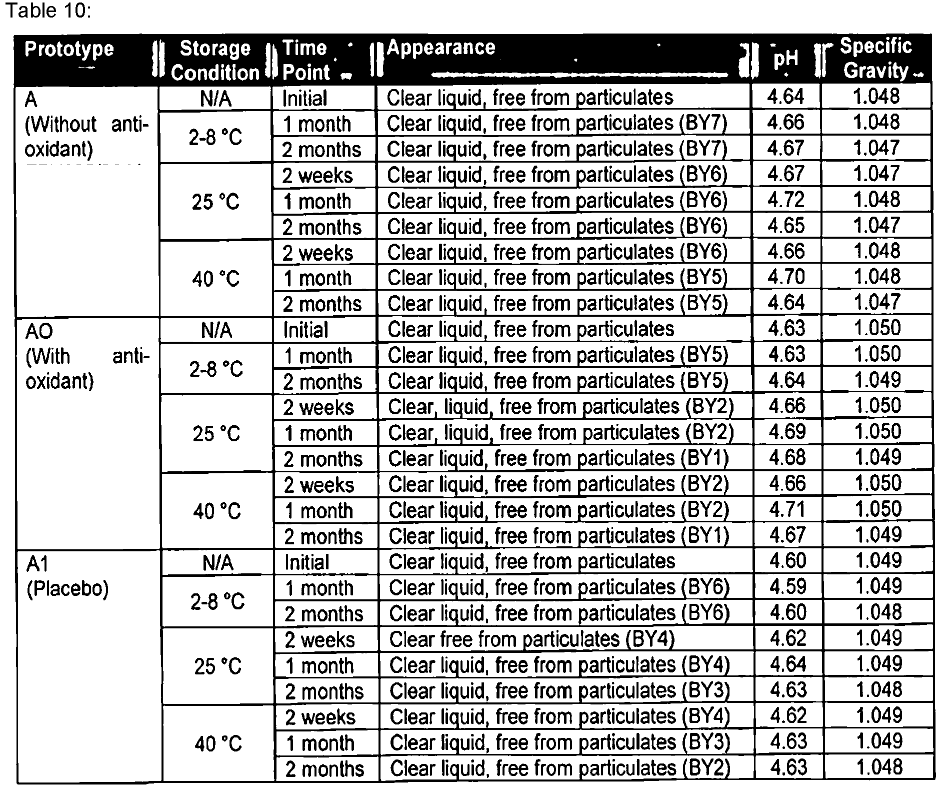

Table 10 also summarizes the results of the appearance in color of the prototypes stored for 2 weeks, 1 month, or 2 months as indicated, and at temperatures of 2-8°C, 25°C, or 40°C. Color change was assessed visually and quantified using the European Pharmacopoeia Color Standard (see "BY" references with lower numbers representing a darker hue). Based on the results in Table 10, Prototype A (without antioxidant) did not discolor as much as Prototype AO (with antioxidant) or Prototype A1 (placebo), especially at the higher temperatures used. Prototype A (without antioxidant) did have some discoloration at 40°C, but there was only a slight color change at 25°C, and no color change at 2-8°C. Notably, the Prototype A1 (placebo) did not discolor as much as Prototype AO (with antioxidant) indicating that discoloration was due at least in part to the presence of the antioxidant (sodium ascorbate). The discoloration of solutions containing sodium ascorbate was not unprecedented; however, the speed of the discoloration was unexpected as a color change was visible after only 2 weeks in Prototype AO at 25°C.

Table 11 summarizes the results of the chemical characterization of clemizole HCl ("assay") of the prototypes stored for 2 weeks, 1 month, or 2 months as indicated, and at temperatures of 2-8°C, 25°C, or 40°C. As indicated in Table 11, the assay of both Prototype A and Prototype AO was low initially. It is believed that this may have been due to sieving, and therefore loss of the clemizole HCl, after dispensing it during the manufacture of the samples. However, the assay did not change from the initial time point for any of the samples, regardless of storage and condition.

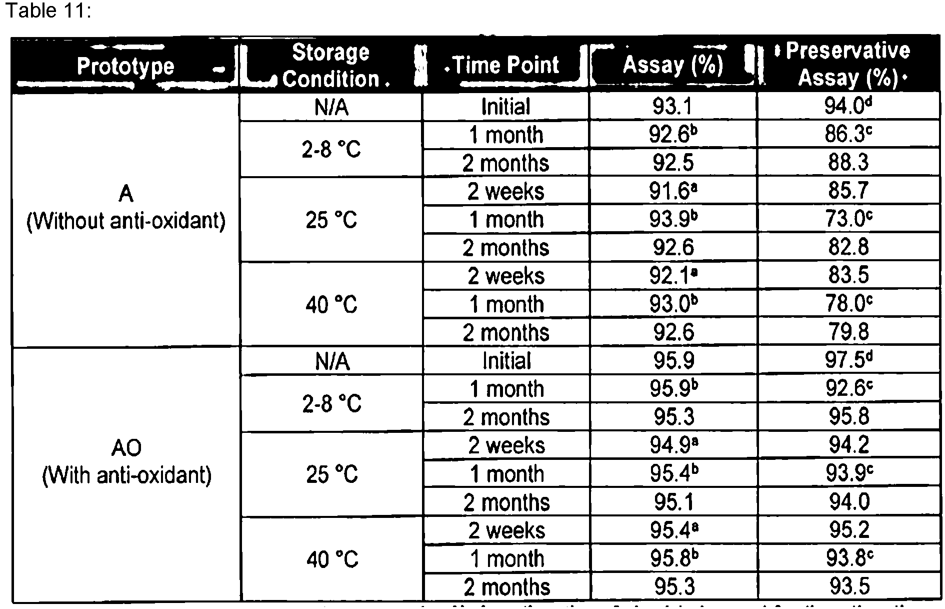

Table 11 also summarizes the results of the chemical characterization of the preservatives ("preservative assay"). As indicated in Table 11, the assay of both Prototype A and Prototype AO was low initially as well. The explanation for this is not certain. There was a decrease over time and temperature for both Prototype A and Prototype AO; however, the decrease was less when the antioxidant was present. For example, Prototype AO (without antioxidant) stored at 25°C decreased by 3.5% from the initial assay compared with an 11% decrease for Prototype A (without antioxidant).Upon review of the literature it was noted that potassium sorbate can undergo oxidation to form carbonyl moieties, and that this is accelerated at higher temperatures. Therefore, it was not surprising that an antioxidant helped to prevent the loss of preservative.

Table 12 summarizes the results of the chemical characterization of related substances of the prototypes stored for 2 weeks, 1 month, or 2 months as indicated, and at temperatures of 2-8°C, 25°C, or 40°C. The amount of related substances does not change regardless of storage time and temperature from the initial time point indicating that there is no impurity increase on stability. In fact, the only real impurity recorded was at relative retention time (RRT) 0.40-0.41, and it is present at a low level (0.06%).

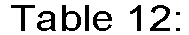

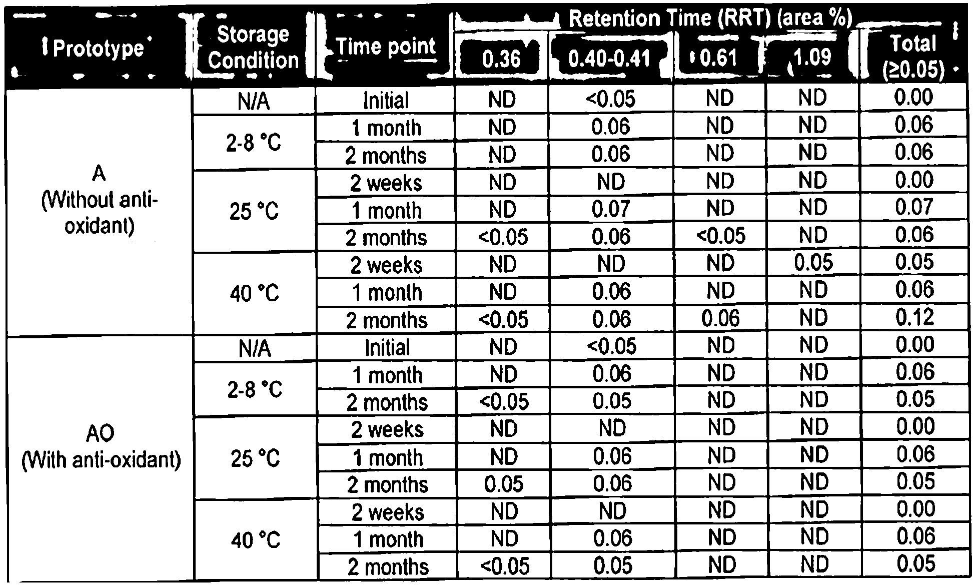

The syringe compatibility and dose uniformity of Prototype A and Prototype AO were evaluated. Two different syringes were used: an Adelphi "Elm Tip," code 706100 (6 ml short form dosing pipette), and an Adelphi Flat Tip, code 90500 (oral dosing pipette). There was no real change in clemizole HCl assay for either Prototype A or Prototype AO in either syringe over 24 hours suggesting that each syringe type is compatible with both formulations. The "related substances" data also confirms the compatibility with either syringe type.

For delivered dose from a multi-purpose container both syringes met the criteria set according to Ph.Eur. 2.9.27 indicating that either syringe was compatible with delivering both Prototype A and Prototype AO formulations accurately. Table 13 illustrates the delivery data for the accuracy in delivering 20, 1 ml doses.

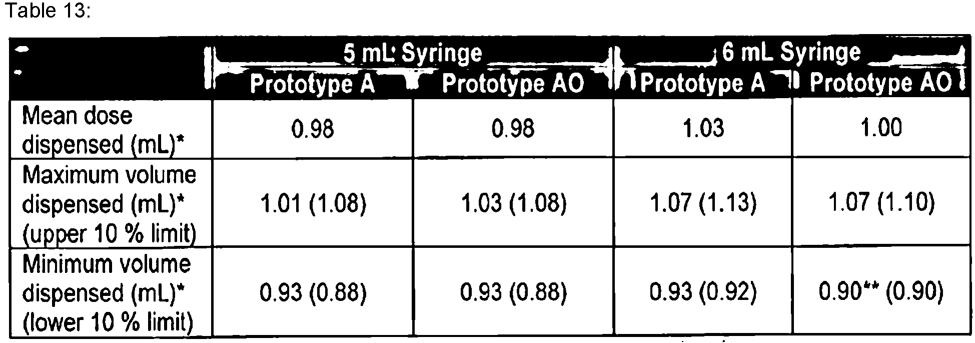

### Example 8:

Formulations other than Prototypes A, AO, B, and BO were also developed. Given the formation of a precipitate upon interactions between the parabens (sodium methyl parahydroxybenzoate and sodium ethyl parahydroxybenzoate) and clemizole HCl observed in Prototype B and Prototype BO of Example 6, other formulations were considered that varied the type of preservative used, the amount of preservative used, the presence or not of an anti-oxidant, and the presence or not of a solubilizer. Example formulations C - K are provided below in Table 14.

Alarm limits for any potential changes to stability may include at least one of the following: regarding appearance, any change from initial appearance; regarding formation of related substances, +/- 5% change from nominal or any individual impurity above 0.05%; regarding preservative stability, +/- 5% change from nominal; regarding specific gravity, +/- 2% change from initial; and regarding pH, any change greater than 0.2 units from initial, wherein potential changes will be monitored at sampling intervals.

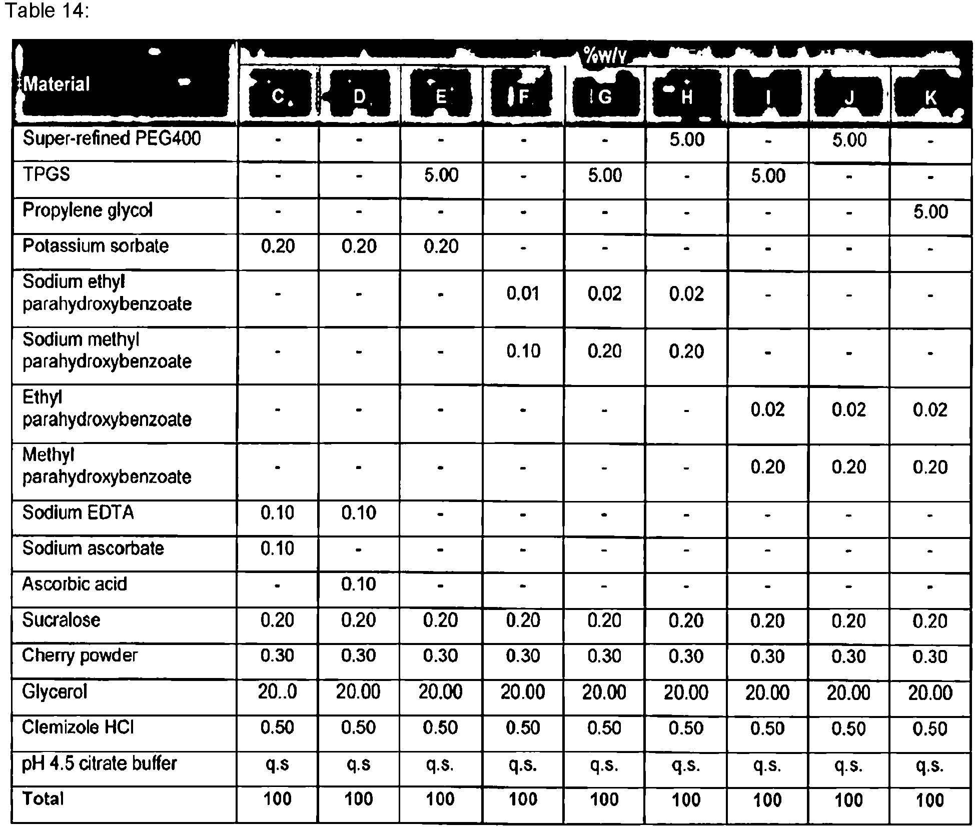

### Example 9:

In an example, the preservative used was potassium sorbate. EDTA along with sodium ascorbate were added, as represented by Formulation C. Formulation C was discontinued due to the formation of particulates observed following the addition of clemizole HCl.

### Example 10:

In an example, the preservative used was sodium methyl parahydroxybenzoate and sodium ethyl parahydroxybenzoate, as represented by Formulation F. Formulation F was discontinued due to the formation of a precipitate observed following the addition of sodium methyl parahydroxybenzoate.

### Example 11:

In an example, the preservative used was sodium methyl parahydroxybenzoate and sodium ethyl parahydroxybenzoate, both at twice the concentration to Formulation F. PEG400 was also added, as represented by Formulation H. Formulation H was discontinued due to the formation of a precipitate observed following the addition of sodium methyl parahydroxybenzoate.

### Example 12:

In an example, the preservative used was sodium methyl parahydroxybenzoate and sodium ethyl parahydroxybenzoate, both at twice the concentration to Formulation F. Propylene glycol was also added, as represented by Formulation K. Formulation K was discontinued due to the formation of a brown sticky precipitate observed following the addition of propylene glycol.

### Example 13:

In an example, the preservative used was potassium sorbate. EDTA along with ascorbic acid were added, as represented by Formulation D. Preparation of Formulation D did not result in the formation of particulates or precipitate, Formulation D was assessed for appearance testing and quantified using the European Pharmacopoeia Color Standard as described below in Table 15. The samples remained clear, and colorless to very pale-yellow following formation of the formulation. However, following storage for 1 month, the samples darkened to BY3/BY4 when storages was performed at 2-8°C and darkened to greater than B1 and BY1 when storages was performed at 25°C, or 40°C. Formulation D was discontinued.

### Example 14:

In an example, the preservative used was potassium sorbate. The combined antioxidant-solubilizer TPGS is added as represented by Formulation E.

Formulation E was assessed for appearance testing and quantified using the European Pharmacopoeia Color Standard as described below in Table 15. Formulation E was pale yellow and lacked the formation of a precipitate. Accordingly, stability testing was performed at both 1 month and 2 months of storage at 2-8°C, 25°C, and 40°C. Formulation E showed a slight color change following storage, which was similar under all storage conditions.

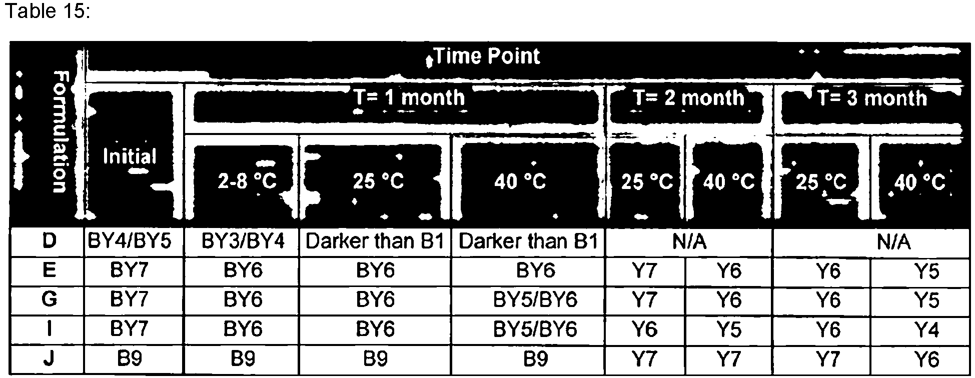

The pH value of Formulation E remained close to 4.5 throughout all storage conditions as illustrated in Table 16 below.

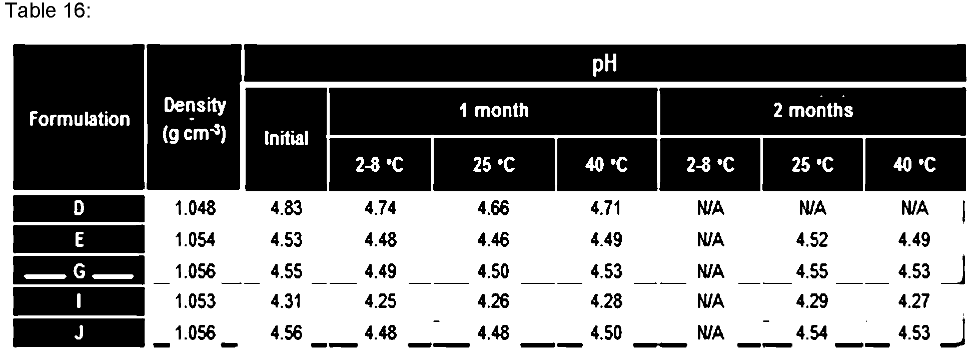

Table 17 below summarizes the results of the chemical characterization of clemizole HCl ("assay") of the formulations stored for 1 month or 2 months as indicated, and at temperatures of 2-8°C, 25°C, and 40°C. As indicated in Table 17, the assay of Formulation E did not change from the initial time point for any of the samples, regardless of storage time or conditions.

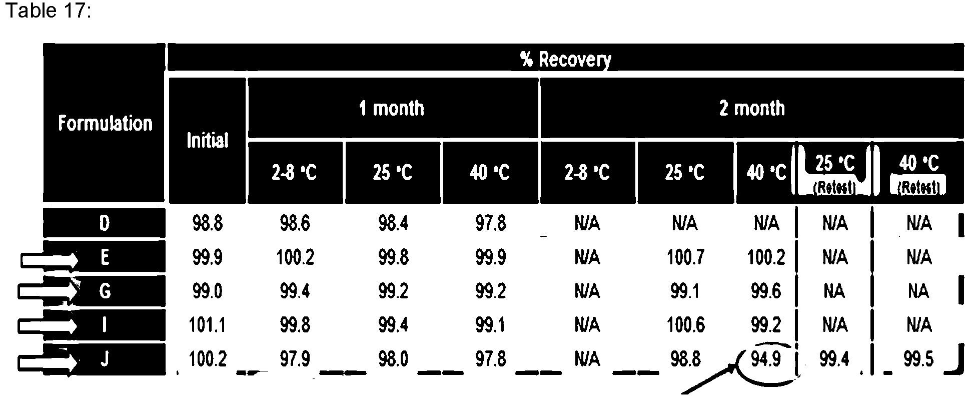

As illustrated below in Table 19, there was a slight increase of related substances in following both 1 and 2 months of storage at 40°C with Formulation E, as well as following 2 months of storage at 25°C. RRT values ranged between 0.61 and 1.56.

Table 18 below summarizes the results of the chemical characterization of the preservatives ("preservative assay") following 1 month, 2 months, and 3 months of storage at temperatures of 2-8°C, 25°C, and 40°C. As indicated in Table 18, the assay for potassium sorbate did not demonstrate any significant loss greater than 5% at all of the time points tested and at all temperatures.

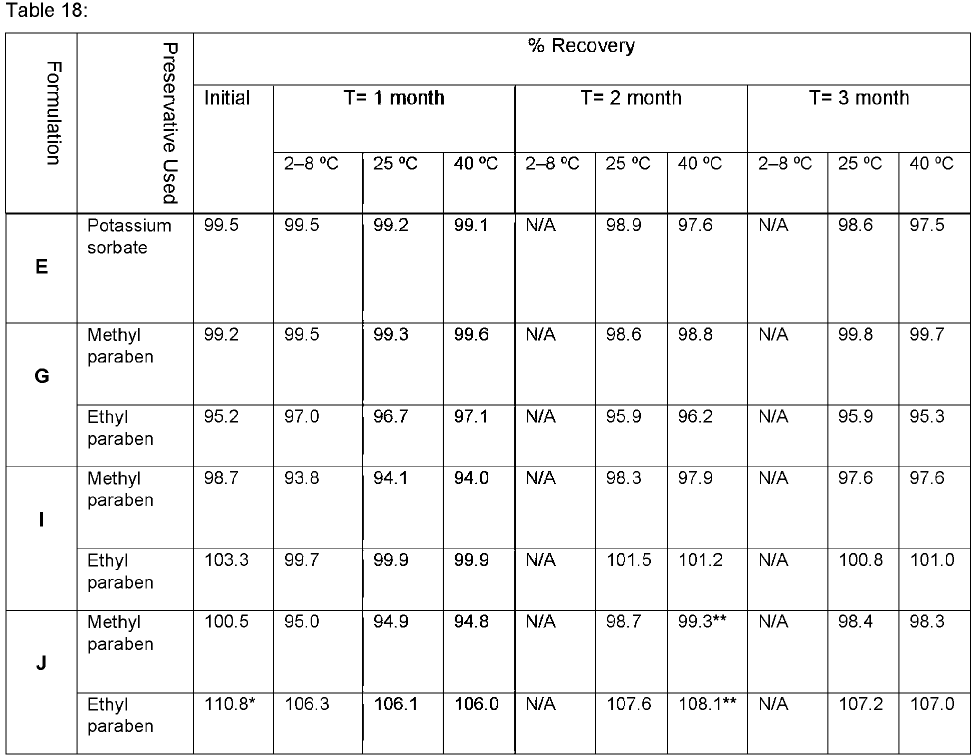

### Example 15:

In an example, the preservative used is sodium methyl parahydroxybenzoate and sodium ethyl parahydroxybenzoate in the amounts indicated in Table 14. The combined antioxidant-solubilizer TPGS was added as represented by Formulation G. Formulation G was assessed for appearance testing and quantified using the European Pharmacopoeia Color Standard as described above in Table 15. Formulation G was pale yellow and lacked the formation of a precipitate. Accordingly, stability testing was performed at both 1 month and 2 months of storage at 2-8°C, 25°C, and 40°C. Formulation G showed a slight color change mostly during storage at 40°C.

The pH value of Formulation G remained close to 4.5 throughout all storage conditions as illustrated above in Table 16.

Table 17 above summarizes the results of the chemical characterization of clemizole HCl ("assay") of the formulations stored for 1 month or 2 months as indicated, and at temperatures of 2-8°C, 25°C, and 40°C. As indicated in Table 17, the assay of Formulation G did not change from the initial time point for any of the samples, regardless of storage time or condition.

As illustrated below in Table 19, there was no increase of related substances at 1 month following storage at 40°C or following 2 months of storage at 25°C with Formulation G. A slight increase of related substances following 2 months of storage at 40°C was observed. RRT values ranged between 0.61 and 1.56.

Table 18 above summarizes the results of the chemical characterization of the preservatives ("preservative assay") following 1 month, 2 months, and 3 months of storage at temperatures of 2-8°C, 25°C, and 40°C. As indicated in Table 18, the assay for sodium methyl parahydroxybenzoate and sodium ethyl parahydroxybenzoate did not demonstrate any significant loss greater than 5% at all of the time points tested and at all temperatures, with values for the sodium methyl parahydroxybenzoate being slightly higher than those of the sodium ethyl parahydroxybenzoate.

### Example 16:

In an example, the preservative used was methyl parahydroxybenzoate and ethyl parahydroxybenzoate in the amounts indicated in Table 14. The combined antioxidant-solubilizer TPGS was added as represented by Formulation I. Formulation I was assessed for appearance testing and quantified using the European Pharmacopoeia Color Standard as described above in Table 15. Formulation I was pale yellow and lacked the formation of a precipitate. Accordingly, stability testing was performed at both 1 month and 2 months of storage at 2-8°C, 25°C, and 40°C. Formulation I showed a slight color change mostly during storage at 40°C.

The pH value of Formulation I remained close to 4.5 throughout all storage conditions as illustrated above in Table 16.

Table 17 above summarizes the results of the chemical characterization of clemizole HCl ("assay") of the formulations stored for 1 month or 2 months as indicated, and at temperatures of 2-8°C, 25°C, and 40°C. As indicated in Table 17, the assay of formulation I did not change from the initial time point for any of the samples, regardless of storage and condition.

As illustrated below in Table 19, there was a slight increase of related substances following both 1 and 2 months of storage at 40°C with formulation I, as well as following 2 months of storage at 25°C. RRT values ranged between 0.61 and 1.56.

Table 18 above summarizes the results of the chemical characterization of the preservatives ("preservative assay") following 1 month, 2 months, and 3 months of storage at temperatures of 2-8°C, 25°C, and 40°C. As indicated in Table 18, the assay for methyl parahydroxybenzoate and ethyl parahydroxybenzoate did not demonstrate any significant loss greater than 6% at all of the time points tested and at all temperatures, with values for the ethyl parahydroxybenzoate being slightly higher than those of the methyl parahydroxybenzoate. A slight drop in the preservative assay value was observed for methyl parahydroxybenzoate at the 1-month time point, but this appears to recover at the 2-month time point across all temperatures used.

### Example 17:

In an example, the preservative used was methyl parahydroxybenzoate and ethyl parahydroxybenzoate in the amounts indicated in Table 14. The solubilizer PEG400 was added without the presence of an antioxidant as represented by Formulation J. Formulation J was assessed for appearance testing and quantified using the European Pharmacopoeia Color Standard as described above in Table 15. Formulation J showed the least color change over the 3-month storage period. The appearance remained consistent at 1-month even at 40°C while slight changes in appearance were observed following storage at 2-3 months.

The pH value of Formulation J remained close to 4.5 throughout all storage conditions as illustrated above in Table 16.

Table 17 above summarizes the results of the chemical characterization of clemizole HCl ("assay") of the formulations stored for 1 month or 2 months as indicated, and at temperatures of 2-8°C, 25°C, and 40°C. As indicated in Table 17, the assay of Formulation J did not change from the initial time point for most of the samples, regardless of storage and condition, except for the drop observed following 2 months of storage at 40°C. Because this drop was not accompanied by a corresponding increase in related substances (Table 19 below), the sample was re-tested. As indicated in table 17, the results suggest that Formulation J did not change from the initial time point up until 2-months following storage at either 25 °C or 40 °C.

As illustrated below in Table 19, this decrease in clemizole HCl was not accompanied by a corresponding increase in related substances. The increase of related substances of Formulation J following 2 months of storage at 40°C had an RRT value of 0.61.

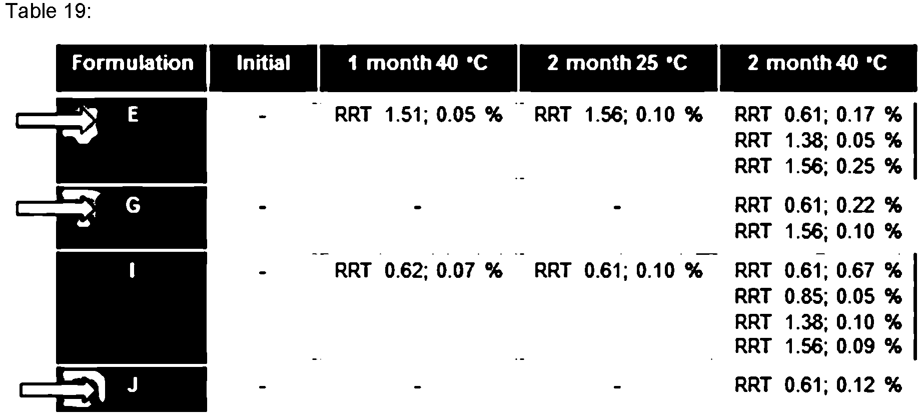

Table 18 above summarizes the results of the chemical characterization of the preservatives ("preservative assay") following 1 month, 2 months, and 3 months of storage at temperatures of 2-8°C, 25°C, and 40°C. As indicated in Table 18, the assay for methyl parahydroxybenzoate and ethyl parahydroxybenzoate did not demonstrate any significant loss greater than 5% at all of the time points tested and at all temperatures, with values for the ethyl parahydroxybenzoate being slightly higher than those of the methyl parahydroxybenzoate. In fact, the values for the ethyl parahydroxybenzoate were slightly higher than target, perhaps due to a slightly higher weight than target that was added (0.109 g versus 0.100 g). A slight drop in the preservative assay value was observed for methyl parahydroxybenzoate at the 1-month time point, but this appeared to recover at the 2-month time point across all temperatures used.

### Example 18:

In an example, taste testing of Formulations E and J, both with either placebo or spiked with bitrex, was conducted. Four participants evaluated the taste of each of the formulations on a scale of 1 to 9, with 1=like extremely and 9=dislike extremely. Results are illustrated below in Table 20 and suggest that either formulation have acceptable taste.

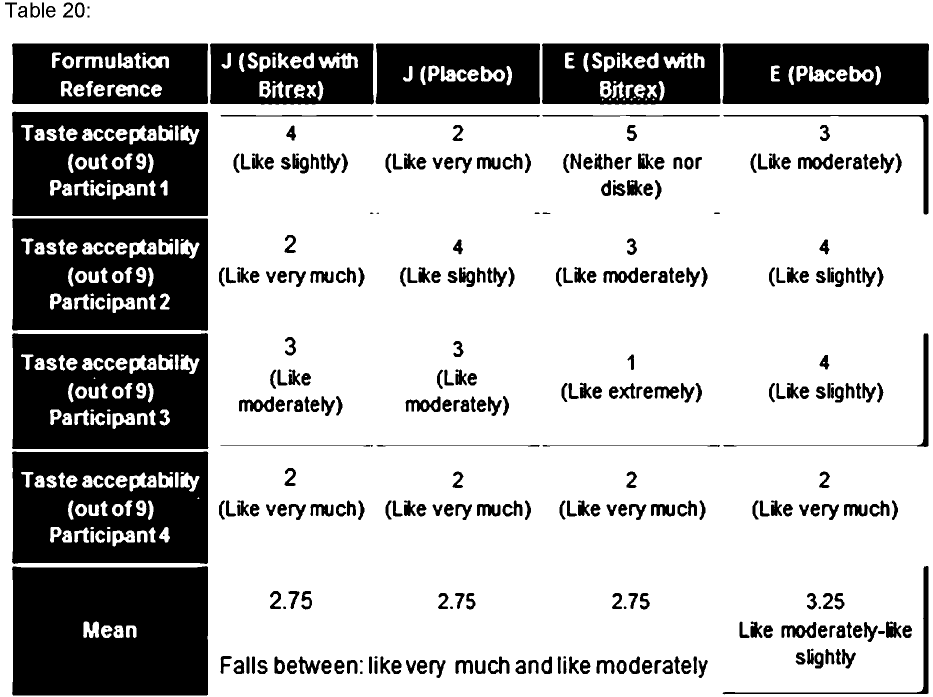

### Example 19:

In an example, Formulation J was again prepared and analyzed following storage at 2 - 8 °C at 60% relative humidity (RH) and 25 °C at 60% RH for up to 36 months, and at 40 °C at 75% RH for up to 6 months. Stability of the formulation was analyzed at 1 month, 3 months, 6 months, 9 months, 12 months, 18 months, 24 months, and 36 months by assessing appearance, activity of clemizole HCl (active assay), formation of related substances to clemizole HCl, preservative stability, pH, and microbial presence, as well as density and fill volume in some examples.

Alarm limits for any potential changes to stability included at least one of the following: regarding appearance, any change from initial appearance; regarding active assay, replicates must agree within 3% of each other and any change from the initial mean must be within 5%, regarding confirmation of clemizole HCl or preservatives any failure to meet specification based on the retention time of the main peak in a sample chromatogram matching that of the main peak in the reference standard chromatogram; regarding formation of related substances, an increase in any single related substance of 0.1% or more compared to previous time points; regarding preservative stability replicates must be within 3% of each other and any changes from initial mean time point greater the 5%; regarding specific gravity, read and record, wherein potential changes will be monitored at sampling intervals; regarding density, read and record, wherein potential changes will be monitored at sampling intervals; regarding pH, read and record, wherein potential changes will be monitored at sampling intervals; regarding microbial limits, failure to meet specification of TAMC ≤10³ CFU/ml, TYMC ≤10² CFU/ml, and absence of *E. coli.*

Formulation J was assessed for appearance testing and quantified using the European Pharmacopoeia Color Standard as described below in Table 21. Formulation J showed a slight color change, mostly following 3 months of storage at 40°C, which returned to a colorless appearance at 6 months, 9 months, 12 months, 18 months, 24 months, and 36 months following storage at temperature 2 - 8 °C and 25 °C, and at 6 months following storage at 40 °C. The appearance of samples under all storage conditions complied with the specification from the initial time point to the 36-month storage time point at all storage temperatures.

**Table 21:**

| Storage Condition | Specification | Time Point | Result |
|---|---|---|---|
| Initial | Clear, colourless to pale yellow solution | Initial | Clear, pale yellow solution |
| | | 1 Month | Clear, pale yellow solution³ |
| | | 3 Months | Clear, pale yellow solution³ |
| | Clear, colourless to pale yellow solution | 6 Months | Clear, pale yellow solution³ |
| 2 - 8 °C | | 9 Months | Clear, pale yellow solution³ |
| | | 12 Months | Clear, pale yellow solution³ |
| | | 18 Months | Clear, pale yellow solution³ |
| | | 24 Months | Clear, pale yellow solution³ |
| | | 36 Months | Clear, pale yellow solution³ |
| | | 1 Month | Clear, pale yellow solution³ |
| | | 3 Months | Clear, pale yellow solution³ |
| | | 6 Months | Clear, pale yellow solution³ |
| 25 °C / 60% RH | Clear, colourless to pale yellow solution | 9 Months | Clear, pale yellow solution³ |
| | | 12 Months | Clear, pale yellow solution³ |
| | | 18 Months | Clear, pale yellow solution³ |
| | | 24 Months | Clear, pale yellow solution³ |
| | | 36 Months | Clear, pale yellow solution³ |
| | | 1 Month | Clear, pale yellow solution³ |
| 40 °C / 75% RH | Clear, colourless to pale yellow solution | 3 Months | Clear, pale yellow solution³ |
| | | 6 Months | Clear, pale yellow solution³ |

The pH value of Formulation J remained close to 4.5 throughout all storage conditions as illustrated in Table 22. The pH values ranged between 4.45 - 4.54. No change in pH was detected outside of normal analytical variability for the samples from the initial time point to the 36-month time point for all storage conditions. Storage temperature and duration of storage did not appear to have an impact on the pH of the formulation.

**Table 22:**

| Storage Condition | Specification | Time Point (Months) | Mean Result |
|---|---|---|---|
| Initial | Read and record | Initial | 4.52 |
| | | 1 Month | 4.53 |
| | | 3 Months | 4.54 |
| | | 6 Months | 4.51⁷ |
| 2 - 8 °C | Read and record | 9 Months | 4.46 |
| | | 12 Months | 4.48 |
| | | 18 Months | 4.52 |
| | | 24 Months | 4.54 |
| | | 36 Months | 4.48 |
| | | 1 Month | 4.50 |
| | | 3 Months | 4.53 |
| | | 6 Months | 4.49⁷ |
| 25 °C / 60% RH | Read and record | 9 Months | 4.45 |
| | | 12 Months | 4.46 |
| | | 18 Months | 4.50 |
| | | 24 Months | 4.52 |
| | | 36 Months | 4.43 |
| | | 1 Month | 4.50 |
| 40 °C /75% RH | Read and record | 3 Months | 4.52 |
| | | 6 Months | 4.47⁷ |

Table 23 below summarizes the results of the chemical characterization of clemizole HCl ("assay") of the formulations stored for 1 month, 3 months, 6 months, 9 months, 12 months, 18 months, 24 months, and 36 months as indicated, and at temperatures of 2 - 8°C, 25°C, and 40°C. The activity of clemizole HCl complied with the specification at all time point measured. The initial mean clemizole HCl activity was 98.3%. Activity remained consistent from the initial time point to the 36-month time point for the 2 - 8°C and 25°C storage conditions, and from the initial time point to the 6-month time point for the 40°C storage condition. The lowest reported mean of clemizole HCl activity was reported at 97.3%, recorded following 6 months of storage at 40 °C.

**Table 23:**

| Storage Condition | Specification | Time Point (Months) | Result (%) |
|---|---|---|---|
| Initial | 90.0-110.0% | Initial | Rep 1: 98.2 |
| | | | Rep 2: 98.5 |
| | | | Mean: 98.3 |
| | | 1 Month | Rep 1: 98.5 |
| | | | Rep 2: 98.9 |
| | | | Mean: 98.7 |
| | | 3 Months | Rep 1: 97.9 |
| | | | Rep 2: 98.2 |
| | | | Mean: 98.0⁴ |
| | | 6 Months | Rep 1: 98.0 |
| | | | Rep 2: 98.5 |
| | | | Mean: 98.3 |
| 2 - 8 °C | 90.0-110.0% | 9 Months | Rep 1: 98.5 |
| | | | Rep 2: 98.5 |
| | | | Mean: 98.5 |
| | | 12 Months | Rep 1: 98.4 |
| | | | Rep 2: 98.4 |
| | | | Mean: 98.4 |
| | | 18 Months | Rep 1: 98.3 |
| | | | Rep 2: 98.6 |
| | | | Mean: 98.4 |
| | | 24 Months | Rep 1: 98.1 |
| | | | Rep 2: 98.2 |
| | | | Mean: 98.2 |
| | | 36 Months | Rep 1: 98.2 |
| | | | Rep 2: 98.3 |
| | | | Mean: 98.3 |
| | | 1 Month | Rep 1: 98.9 |
| | | | Rep 2: 96.9 |
| | | | Mean: 97.9 |
| | | 3 Months | Rep 1: 97.1 |
| | | | Rep 2: 98.0 |
| | | | Mean: 97.6¹ |
| | | 6 Months | Rep 1: 97.7 |
| | | | Rep 2: 98.1 |
| | | | Mean: 97.9 |
| 25 °C / 60% RH | 90.0-110.0% | 9 Months | Rep 1: 98.5 |
| | | | Rep 2: 98.5 |
| | | | Mean: 98.5 |
| | | 12 Months | Rep 1: 98.4 |
| | | | Rep 2: 98.9 |
| | | | Mean: 98.6 |
| | | 18 Months | Rep 1: 98.3 |
| | | | Rep 2: 98.5 |
| | | | Mean: 98.4 |
| | | 24 Months | Rep 1: 97.6 |
| | | | Rep 2: 97.8 |
| | | | Mean: 97.7 |
| | | 36 Months | Rep 1: 97.9 |
| | | | Rep 2: 97.9 |
| | | | Mean: 97.9 |
| | | 1 Month | Rep 1: 99.0 |
| | | | Rep 2: 97.8 |
| | | | Mean: 98.4 |
| 40 °C / 75% RH | 90.0-110.0% | 3 Months | Rep 1: 97.4 |
| | | | Rep 2: 97.4 |
| | | | Mean: 97.4 |
| | | 6 Months | Rep 1: 97.4 |
| | | | Rep 2: 97.1 |
| | | | Mean: 97.3 |

As illustrated below in Table 24, no individual related substances were detected above the reporting limit from the initial time point to the 36-month time point, with a few exceptions. At 25°C a single related substance was detected at 18 months, 24 months, and 36 months at 0.09%, 0.09%, and 0.65%, respectively. At 40°C a single related substance was detected at 3 months and 6 months at 0.09% and 0.20%, respectively. Total related species levels were not above the reporting limit from the initial time point to the 36-month time point with few exceptions. At 25°C, total related substance at 36 months increased to 0.14%, reaching the alarm limit of ≥ 0.1 %. At 40°C, total related substance at 6 months increased again to 0.20%, reaching the alarm limit of ≥ 0.1%. The total amount of related substances formed during storage under all temperatures and at all time points measured were not above the allowed threshold of 3.0% area, nor were any individual impurities measured above the allowed threshold of 0.5% area.

**Table 24:**

| Storage Condition | Specification | Time Point (Months) | Result (%) |
|---|---|---|---|
| Initial | NMT 0.5% area of individual impurities | Initial | RRT 0.85: <0.05 |
| | | | RRT 1.38: <0.05 |
| | NMT 3.0% total impurities | | Total: <0.05 |
| | Report all impurities greater than or equal to 0.05% area | | |
| | | 1 Month | ND |
| | | 3 Months | RRT 1.33 <0.05 |
| | | | Total <0.05 |
| 2 - 8 °C | NMT 0.5% area of individual impurities | 6 Months | RRT 0.42: <0.05 |
| | | | RRT 0.63: <0.05 |
| | NMT 3.0% total impurities | | RRT 0.87: <0.05 |
| | | | RRT 1.40: <0.05 |
| | | | Total: <0.05 |
| | Report all impurities greater than or equal to 0.05% area | | |
| | | 9 Months | RRT 0.37: <0.05 |
| | | | RRT 0.43: <0.05 |
| | | | RRT 0.74: <0.05 |
| | | | RRT 0.86: <0.05 |
| | | | RRT 1.25: <0.05 |
| | | | RRT 1.39: <0.05 |
| | | | Total: <0.05 |
| | | 12 Months | RRT 0.36: <0.05 |
| | | | RRT 0.42: <0.05 |
| | | | RRT 0.86: <0.05 |
| | | | RRT 1.39: <0.05 |
| | | | Total: <0.05 |
| | | 18 Months | RRT 0.40: <0.05 |
| | | | RRT 0.85: <0.05 |
| | | | RRT 1.31: <0.05 |
| | | | Total: <0.05 |
| | | 24 Months | RRT 0.33: <0.05 |
| | | | RRT 0.40: <0.05 |
| | | | RRT 0.85: <0.05 |
| | | | RRT 1.37: <0.05 |
| | | | Total: <0.05 |
| | | 36 Months | Non detected above LOQ |
| | | 1 Month | ND |
| | | 3 Months | RRT 1.33: <0.05 |
| | | | Total: <0.05 |
| | | 6 Months | RRT 0.42: <0.05 |
| | | | RRT 0.63: <0.05 |
| | | | RRT 0.87: <0.05 |
| | | | RRT 1.19: <0.05 |
| | | | RRT 1:40: <0.05 |
| | | | Total: <0.05 |
| 25 °C / 60% RH | NMT 0.5% area of individual impurities | 9 Months | RRT 0.36: <0.05 |
| | | | RRT 0.42: <0.05 |
| | NMT 3.0% total impurities | | RRT 0.63: <0.05 |
| | | | RRT 0.86: <0.05 |
| | | | RRT 1.25 <0.05 |
| | | | RRT 1.39 <0.05 |
| | Report all impurities greater than or equal to 0.05% area | | Total: <0.05 |
| | | 12 Months | RRT 0.36: <0.05 |
| | | | RRT 0.42: <0.05 |
| | | | RRT 0.63: <0.05 |
| | | | RRT 0.86: <0.05 |
| | | | RRT 1.39: <0.05 |
| | | | Total: <0.05 |
| | | 18 Months | RRT 0.40: <0.05 |
| | | | RRT 0.49: <0.05 |
| | | | RRT 0.60: 0.09 |
| | | | RRT 0.85: <0.05 |
| | | | RRT 1.31: <0.05 |
| | | | Total: 0.09 |
| | | 24 Months | RRT 0.33: <0.05 |
| | | | RRT 0.40: <0.05 |
| | | | RRT 0.60: 0.09 |
| | | | RRT 0.85: <0.05 |
| | | | RRT 1.27: <0.05 |
| | | | RRT 1.37: <0.05 |
| | | | Total: 0.09 |
| | | 36 Months | RRT 0.65: 014 |
| | | | Total: 0.14 |
| | | 1 Month | ND |
| 40 °C / 75% RH | NMT 0.5% area of individual impurities | 3 Months | RRT 0.62: 0.09 |
| | | | RRT 1.33: <0.05 |
| | NMT 3.0% total impurities | | Total: 0.09 |
| | Report all impurities greater than or equal to 0.05% area | | |
| | | 6 Months | RRT 0.42: <0.05 |
| | | | RRT 0.58: <0.05 |
| | | | RRT 0.63: 0.20 |
| | | | RRT 0.87: <0.05 |
| | | | RRT 1.40: <0.05 |
| | | | Total: 0.20 |

Tables 25 and 26 below summarize the results of the chemical characterization of the preservatives ("preservative assay") following 1 month, 3 months, 6 months, 9 months, 12 months, 18 months, 24 months and 36 months of storage at temperatures of 2 - 8°C, 25°C, and 40°C. The activity assay performed for both the methyl paraben and ethyl paraben preservatives at all time points and under all storage temperature conditions did not decrease beyond the allowed threshold As indicated in Tables 25 and 26, the assay for methyl paraben and ethyl paraben, respectively, did not demonstrate any loss greater than 3.2% at all of the time points tested and at all storage temperatures.

**Table 25: Ethyl Paraben Assay**

| Storage Condition | Specification | Time Point (Months) | Result (%) |
|---|---|---|---|
| Initial | 90.0 - 110.0% | Initial | Rep 1: 98.2 |
| | | | Rep 2: 98.2 |
| | | | Mean: 98.2 |
| | | 1 Month | Rep 1: 97.6 |
| | | | Rep 2: 97.6 |
| | | | Mean: 97.6⁵ |
| | | 3 Months | Rep 1: 97.4 |
| | | | Rep 2: 97.7 |
| | | | Mean: 97.5 |
| | | 6 Months | Rep 1: 97.8 |
| | | | Rep 2: 97.8 |
| | | | Mean: 97.8 |
| 2 - 8 °C | 90.0 - 110.0% | 9 Months | Rep 1: 98.1 |
| | | | Rep 2: 98.2 |
| | | | Mean: 98.2 |
| | | 12 Months | Rep 1: 97.8 |
| | | | Rep 2: 98.0 |
| | | | Mean: 97.9 |
| | | 18 Months | Rep 1: 97.0 |
| | | | Rep 2: 97.0 |
| | | | Mean: 97.0 |
| | | 24 Months | Rep 1: 97.7 |
| | | | Rep 2: 97.6 |
| | | | Mean: 97.7 |
| | | 36 Months | Rep 1: 98.7 |
| | | | Rep 2: 98.7 |
| | | | Mean: 98.7 |
| | | 1 Month | Rep 1: 97.8 |
| | | | Rep 2: 97.8 |
| | | | Mean: 97.8⁵ |
| | | 3 Months | Rep 1: 98.0 |
| | | | Rep 2: 97.9 |
| | | | Mean: 98.0 |
| | | 6 Months | Rep 1: 98.0 |
| | | | Rep 2: 97.7 |
| | | | Mean: 97.9 |
| 25 °C / 60% RH | 90.0 - 110.0% | 9 Months | Rep 1: 98.0 |
| | | | Rep 2: 97.7 |
| | | | Mean: 97.9 |
| | | 12 Months | Rep 1: 97.7 |
| | | | Rep 2: 97.7 |
| | | | Mean: 97.7 |
| | | 18 Months | Rep 1: 96.9 |
| | | | Rep 2: 97.1 |
| | | | Mean: 97.0 |
| | | 24 Months | Rep 1: 96.8 |
| | | | Rep 2: 97.2 |
| | | | Mean: 97.0 |
| | | 36 Months | Rep 1: 98.5 |
| | | | Rep 2: 98.4 |
| | | | Mean: 98.4 |
| | | 1 Month | Rep 1: 97.8 |
| | | | Rep 2: 97.9 |
| | | | Mean: 97.8⁴ |
| | | 3 Months | Rep 1: 97.8 |
| | | | Rep 2: 97.8 |
| | | | Mean: 97.8 |
| 40 °C / 75% RH | 90.0 - 110.0% | 6 Months | Rep 1: 97.4 |
| | | | Rep 2: 97.0 |
| | | | Mean: 97.2 |

**Table 26: Methyl Paraben Assay**

| Storage Condition | Specification | Time Point (Months) | Result (%) |
|---|---|---|---|
| Initial | 90.0 - 110.0% | Initial | Rep 1: 100.0 |
| | | | Rep 2: 99.9 |
| | | | Mean: 100.0 |
| | | 1 Month | Rep 1: 97.9 |
| | | | Rep 2: 98.0 |
| | | | Mean: 97.9⁸ |
| | | 3 Months | Rep 1: 98.4 |
| | | | Rep 2: 98.5 |
| | | | Mean: 98.4 |
| | | 6 Months | Rep 1: 97.0 |
| | | | Rep 2: 97.1 |
| | | | Mean: 97.0 |
| 2 - 8 °C | 90.0 - 110.0% | 9 Months | Rep 1: 98.5 |
| | | | Rep 2: 98.5 |
| | | | Mean: 98.5 |
| | | 12 Months | Rep 1: 97.8 |
| | | | Rep 2: 98.0 |
| | | | Mean: 97.9 |
| | | 18 Months | Rep 1: 99.1 |
| | | | Rep 2: 99.1 |
| | | | Mean: 99.1 |
| | | 24 Months | Rep 1: 98.2 |
| | | | Rep 2: 98.1 |
| | | | Mean: 98.1 |
| | | 36 Months | Rep 1: 97.8 |
| | | | Rep 2: 97.8 |
| | | | Mean: 97.8 |
| | | 1 Month | Rep 1: 98.0 |
| | | | Rep 2: 98.0 |
| | | | Mean: 98.0⁴ |
| | | 3 Months | Rep 1: 98.7 |
| | | | Rep 2: 98.9 |
| | | | Mean: 98.8 |
| | | 6 Months | Rep 1: 97.2 |
| | | | Rep 2: 96.9 |
| | | | Mean: 97.1 |
| | | 9 Months | Rep 1: 98.3 |
| 25 °C / 60% RH | 90.0 - 110.0% | | Rep 2: 98.2 |
| | | | Mean: 98.3 |
| | | 12 Months | Rep 1: 97.8 |
| | | | Rep 2: 97.9 |
| | | | Mean: 97.8 |
| | | 18 Months | Rep 1: 99.0 |
| | | | Rep 2: 99.1 |
| | | | Mean: 99.1 |
| | | 24 Months | Rep 1: 97.4 |
| | | | Rep 2: 97.8 |
| | | | Mean: 97.6 |
| | | 36 Months | Rep 1: 97.5 |
| | | | Rep 2: 97.5 |
| | | | Mean: 97.5 |
| | | 1 Month | Rep 1: 97.7 |
| | | | Rep 2: 97.9 |
| | | | Mean: 97.8⁵ |
| | | 3 Months | Rep 1: 98.8 |
| | | | Rep 2: 98.6 |
| | | | Mean: 98.7 |
| 40 °C / 75% RH | 90.0 - 110.0% | 6 Months | Rep 1: 97.0 |
| | | | Rep 2: 96.6 |
| | | | Mean: 96.8 |

Confirmation of the presence of clemizole HCl, ethyl paraben, and methyl paraben was determined. The retention time of the main peak in sample chromatograms corresponded to that of the main peak in the reference standard chromatogram for each.

All storage conditions evaluated were within the allowed limits of microbial detection at all time points. The detected levels of microbials were TAMC <10 CFU/ml and TYMC <10 CFU/ml. The presence of E. *coli* was not detected.

### Example 20:

In an example, Formulations C and D were prepared as follows. An excess of each solid excipient including cherry flavor, sweetener, antioxidants, and preservative, were sieved through a 500 µM mesh, then dispensed into individual weigh boats or beakers at the required quantity. pH 4.5 citrate buffer was dispensed into a beaker and the excipients were added to the buffer in the order of preservative, antioxidant, sweetener, and flavor. Each excipient was dissolved fully prior to adding the next. Glycerol was then dispensed into the buffer-excipient solution and mixed until the solution appeared homogeneous. Clemizole HCl was then added, ensuring the mixture was homogenous prior to measuring the pH of the resulting solution. If the pH was not within the target range (4.5 +/-0.5), the pH was adjusted within the target by adding 0.5 M citric acid solution or 0.5 M trisodium citrate solution while stirring. The solution was brought to volume with pH 4.5 citrate buffer.

### Example 21:

In an example, Formulation E is prepared as follows. An excess of each solid excipient including cherry flavor, sweetener, and preservative, are sieved through a 500 µM mesh, then dispensed into individual weigh boats or beakers at the required quantity. TPGS was first melted in its container in an oven set at 55°C for 1 hour. In a separate beaker, pH 4.5 citrate buffer was dispensed. While stirring the following excipients were added to the beaker containing buffer in this order, allowing for each component to fully dissolve before adding the next: TPGS, preservative, sweetener, and flavor. Glycerol was then added and mixed until fully homogenous. Next, clemizole HCl was added to the beaker, ensuring the mixture was homogenous. If the pH was not within the target range (4.5 +/-0.5), the pH was adjusted within the target range by adding 0.5 M citric acid solution or 0.5 M trisodium citrate solution while stirring. The solution was brought to volume with pH 4.5 citrate buffer.

### Example 22:

In an example, Formulations F, G, and H were prepared as follows. An excess of each solid excipient including cherry flavor, sweetener, and preservative, were sieved through a 500 µM mesh, then dispensed into individual weigh boats or beakers at the required quantity. TPGS was first melted in its container in an oven set at 55°C for 1 hour. In a separate beaker, pH 4.5 citrate buffer was dispensed. For Formulations G and H, the solubilizer was added to the buffer and mixed until dissolved. For Formulations F, G, and H, clemizole HCl was added to the beaker and mixed until fully dissolved. While stirring, the remaining excipients were added in this order, allowing for each component to fully dissolve before adding the next: sodium ethyl parahydroxybenzoate, sodium methyl parahydroxybenzoate, sweetener, and flavor. Glycerol was then added and mixed until fully homogenous. If the pH was not within the target range (4.5 +/-0.5), the pH was adjusted within the target by adding 0.5 M citric acid solution or 0.5 M trisodium citrate solution while stirring. The solution was brought to volume with pH 4.5 citrate buffer.

### Example 23:

In an example, Formulations F, G, and H were prepared as follows. An excess of each solid excipient including cherry flavor, sweetener, and preservative, were sieved through a 500 µM mesh, then dispensed into individual weigh boats or beakers at the required quantity. TPGS was first melted in its container in an oven set at 55°C for 1 hour. In a separate beaker, solubilizer (TPGS or PEG400) was dispensed. If the solubilizer was TPGS, then the beaker will need to be warmed to approximately 50°C on a hotplate until the sweetener was added. Next, ethyl parahydroxybenzoate and then methyl parahydroxybenzoate were added, ensuring that the first preservative was dissolved before adding the second preservative. pH 4.5 citrate buffer was dispensed into a second beaker. Clemizole HCl was then added and dissolved in the second beaker containing pH 4.5 citrate buffer. While stirring, the contents of the first beaker comprising TPGS and the preservatives were added to the second beaker comprising pH 4.5 citrate buffer and clemizole HCl. The sweetener was added next, followed by the flavor agent (cherry flavor), ensuring that the sweetener was fully dissolved prior to addition of the flavoring agent. Glycerol was then added and mixed until fully homogenous. If the pH was not within the target range (4.5 +/-0.5), the pH was adjusted within the target by adding 0.5 M citric acid solution or 0.5 M trisodium citrate solution while stirring. The solution was brought to volume with pH 4.5 citrate buffer.

Following preparation of the formulations, tests including stability testing, clemizole HCl assay, preservative assay, and appearance could be performed.

### Example 24:

In an example, three formulations were prepared. Each of the three formulations were similar to Formulation J in that the preservative used was methyl parahydroxybenzoate and ethyl parahydroxybenzoate and the solubilizer PEG400 was included. The amounts of preservative, PEG 400, flavor (cherry flavor powder), sweetener (sucralose), glycerol, and citrate buffer components (citric acid monohydrate and trisodium citrate dihydrate) were identical between the individual formulations as illustrated in Table 27 below. The three formulations illustrated in Table 27 included either 5 mg/mL clemizole HCl, 10 mg/mL clemizole HCl, or 20 mg/mL clemizole HCl.

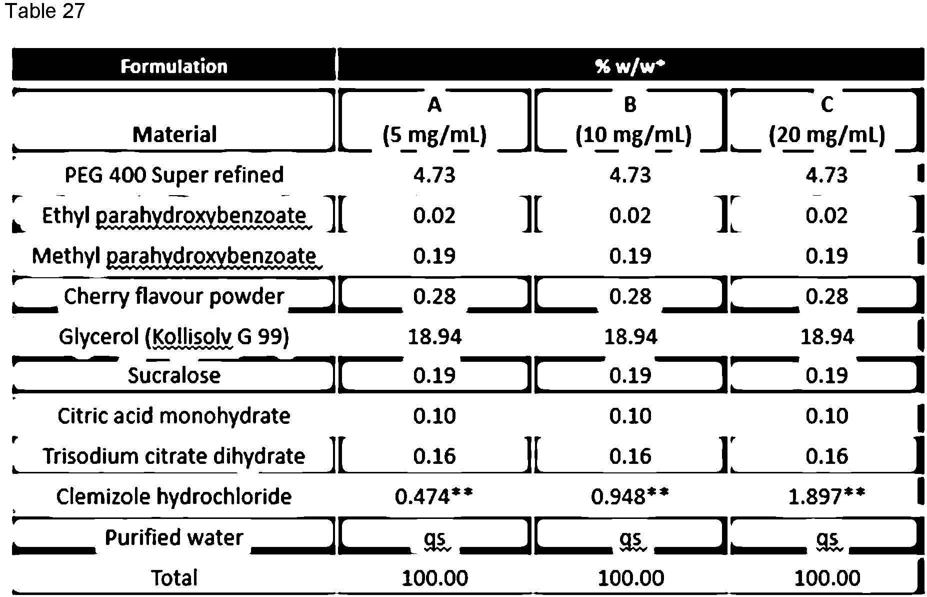

The formulations were assayed for solubility and stability including: appearance (colorless to pale yellow and free from crystalline precipitate), clemizole HCl stability (90.0% to 110.0% recovery), formation of related substances to clemizole HCl (+/- 0.5% change for any individual impurity or 3.0% change for total related substances), preservative stability (+/- 10% change from nominal; 90.0% to 110.0% recovery), and pH (any change greater than 0.2 units from initial).

All three formulations demonstrated a high solubility of clemizole HCl. The 20 mg/mL clemizole HCl formulation failed to solubilize completely. The formulation was filtered and the solubility assay was performed on the remaining portion in solution. Solubility assays for all formulations demonstrated solubilities of 102.5%, 103.8%, and 97.6% for the 5 mg/mL, 10 mg/mL, and 20 mg/mL clemizole HCl samples, respectively.

The appearance of all three formulations were clear, colorless, and free from crystalline precipitate upon formation.

Upon formation, the pH value of the three formulations were close to 4.5 as illustrated below in Table 28.

**Table 28**

| | 5 mg/mL formulation | 10 mg/mL formulation | 20 mg/mL formulation |
|---|---|---|---|
| pH | 4.448 | 4.386 | 4.356 |

The clemizole HCl assay of all three formulations were within the allowed specification limits as illustrated in Table 29 below.

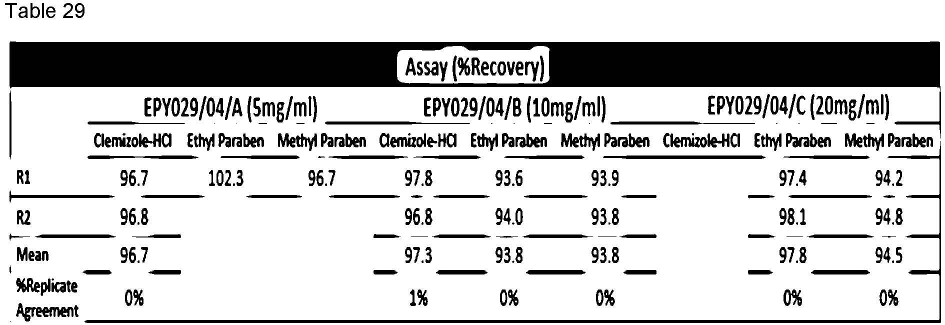

There were no related substances detected in the three formulations.

Preservative assays for the three formulations provided mixed results. Assay results for ethyl parahydroxybenzoate were within the specification limits for all three formulations. Assay results for methyl parahydroxybenzoate for the 5 mg/mL and 20 mg/mL clemizole HCl sample were within specification limits as illustrated below in Table 30. Assay results for methyl parahydroxybenzoate for the 10 mg/mL clemizole HCl sample were lower than specification limits.

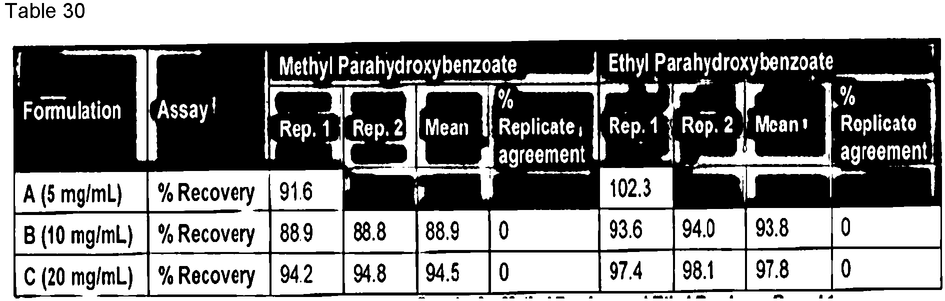

### Example 25:

In an example, three formulations were prepared according to the formulations in Example 24 with the exception of the clemizole HCl concentration as illustrated in Table 31 below. The three formulations included either 10 mg/mL clemizole HCl (formulation B), 16 mg/mL clemizole HCl (formulation D), or 18 mg/mL clemizole HCl (formulation E).

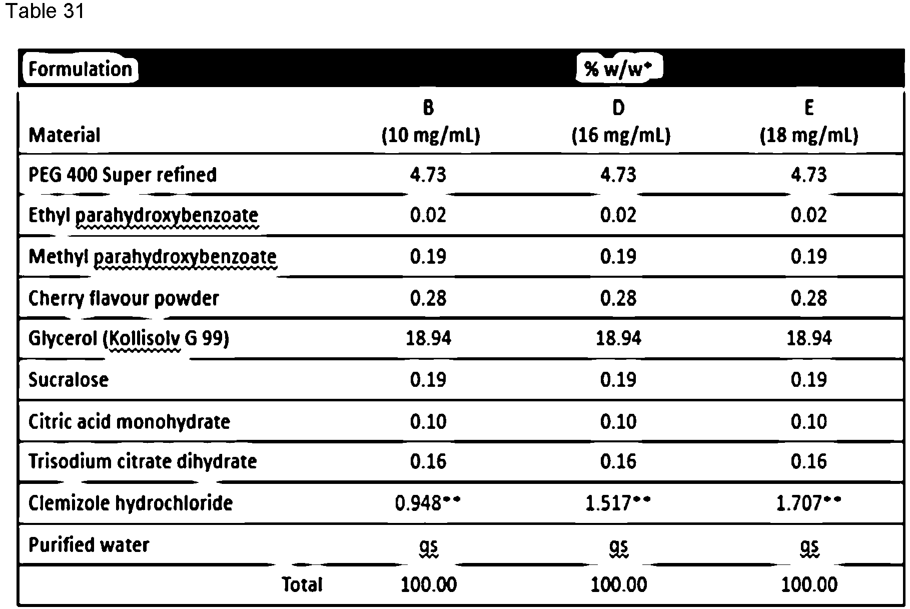

The formulations were assayed for solubility and any potential changes from initial formation to 28 days following storage at 25 °C or 40 °C. Potential changes included: appearance (colorless to pale yellow and free from crystalline precipitate), clemizole HCl stability (assay results between 90.0% to 110.0%), formation of related substances to clemizole HCl (+/- 0.5% change for any individual impurity or 3.0% change for total related substances), preservative stability (+/- 10% change from nominal), and pH (any change greater than 0.1 units from initial).

The formulation comprising 18 mg/mL clemizole HCl (formulation D) failed to solubilize all of the clemizole HCl.

During a first preparation, the formulation comprising 10 mg/mL clemizole HCl (formulation B) demonstrated the formation of lumps of undissolved yellowish solid. The formulation was re-prepared and filtered, and appeared clear, pale yellow, and free from crystalline precipitate. The formulation comprising 16 mg/mL clemizole HCl (formulation D) appeared clear, colorless, homogeneous, and free from crystalline precipitate. The appearance of the 10 mg/mL clemizole HCl (formulation B) and the 16 mg/mL clemizole HCl (formulation D) remained clear, colorless to pale yellow, and free from precipitate during storage at either 25 °C or 40 °C for 7 days, 14 days, and 28 days.

The pH value of the two formulations remained close to 4.5 throughout all storage conditions; however, the results were below the specification limits. The mean pH values ranged between 4.23 and 4.32 throughout storage as illustrated in Table 32 below.

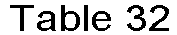

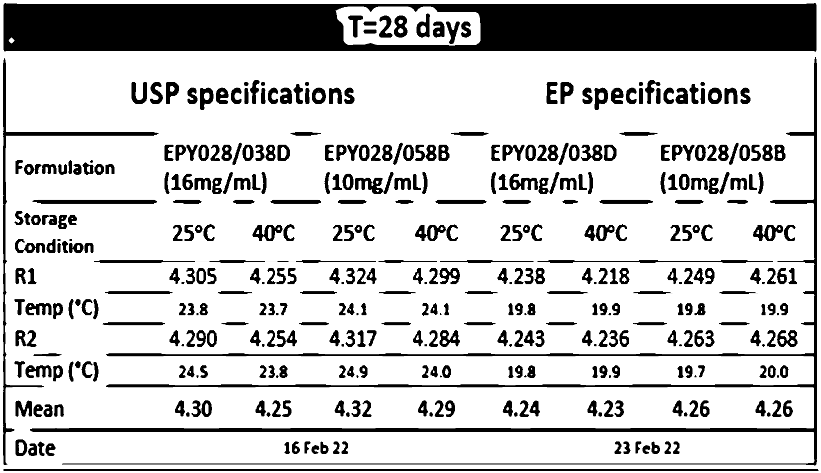

The clemizole HCl assay of the two formulations were within the allowed specification limits throughout all storage conditions as illustrated in Table 33 below.

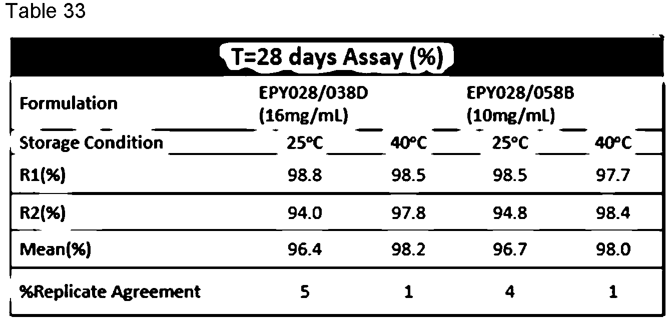

Any formation of related substances were within specification limits throughout all storage conditions as illustrated below in Table 34.

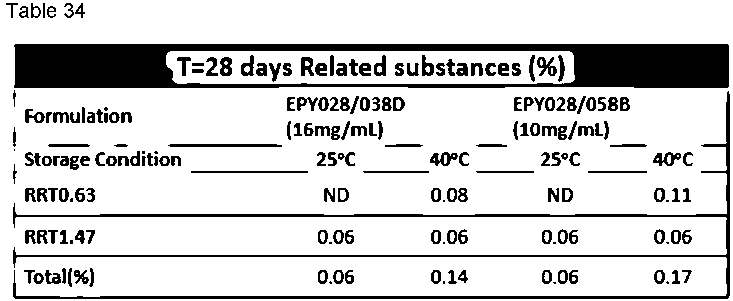

Preservative assays of both ethyl parahydroxybenzoate and methyl parahydroxybenzoate were within the allowed specification limits throughout all storage conditions as illustrated in Table 35 below.

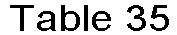

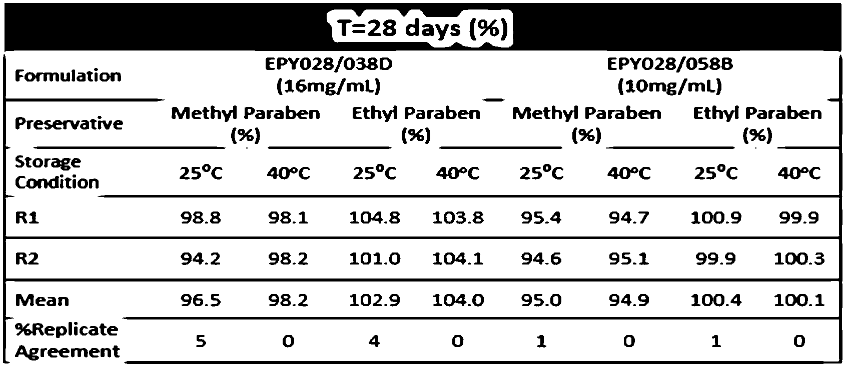

## Claims

1. A liquid pharmaceutical composition comprising
clemizole HCl;
a solvent;
a preservative; and
glycerol, wherein
the preservative comprises potassium sorbate, sodium methyl parahydroxybenzoate, sodium ethyl parahydroxybenzoate, methyl parahydroxybenzoate, or ethyl parahydroxybenzoate,
wherein the solvent comprises a citrate buffer, and wherein
the pH of the liquid pharmaceutical composition ranges from 4 to 5.

2. The liquid pharmaceutical composition of claim 1, wherein the glycerol is at a concentration of 10 - 30% (wt/wt) and the citrate buffer is at a concentration of 90 - 70% (wt/wt).

3. The liquid pharmaceutical composition of claim 1, wherein the glycerol concentration is 20% (wt/wt), and the citrate buffer concentration is 80% (wt/wt).

4. The liquid pharmaceutical composition of any one of the preceding claims,
wherein the preservative comprises methyl parahydroxybenzoate, potassium sorbate, or ethyl parahydroxybenzoate.

5. The liquid pharmaceutical composition of any one of the preceding claims, further comprising a solubilizer, optionally wherein the solubilizer is tocopherol polyethylene glycol succinate (TPGS) or super-refined PEG400.

6. The liquid pharmaceutical composition of any of the preceding claims, further comprising a sweetener and a taste modifier.

7. The liquid pharmaceutical composition of any of the preceding claims, further comprising an anti-oxidant or a component with anti-oxidant properties.

8. The liquid pharmaceutical composition of any of the preceding claims , wherein the clemizole HCl is at a concentration of 1 mg/mL - 30 mg/mL, preferably 5 mg/mL, in the pharmaceutical composition.

9. The liquid pharmaceutical composition of any one of the preceding claims, wherein following up to 36 months of storage at least any one or more of the following occurs:
- there is no substantial increase in clemizole HCl metabolites or related degradation products,
- an amount of the preservative is maintained at substantially 97% or greater of the original amount of the preservative,
- there is no substantial change in pH of the pharmaceutical composition, or
- there is no substantial change in color of the pharmaceutical composition, wherein the storage occurs at a temperature of 2 - 8 °C, 25°C, or 40°C.

10. The liquid pharmaceutical composition of any of the preceding claims, further comprising an anti-epileptic drug.

11. A method of preparing a liquid pharmaceutical composition for treating an epilepsy disorder, comprising:
dissolving at least one of a preservative, clemizole HCl, a plurality of excipients, and glycerol in a solution comprising at least one of a solvent or a solubilizer to form the liquid pharmaceutical composition, wherein
the solvent is a citrate buffer,
the clemizole HCl is at a concentration from 1 mg/mL to 30 mg/mL in the liquid pharmaceutical composition, and
the at least one preservative comprises at least one of potassium sorbate, sodium methyl parahydroxybenzoate, sodium ethyl parahydroxybenzoate, methyl parahydroxybenzoate, or ethyl parahydroxybenzoate,
wherein the method further comprises
adding the solvent when the dissolving is in the solubilizer or adding the solubilizer when the dissolving occurs in the solvent, and
adjusting the pH of the liquid pharmaceutical composition to pH 4.5 (+/- 0.5).

12. The liquid pharmaceutical composition of any one of the preceding claims for use in the treatment of a subject having an epilepsy disorder, wherein the administering comprises one or more routes selected from oral, sublingual, sublabial, buccal, and transmucosal.

13. The liquid pharmaceutical composition for use of claim 12, wherein the dose of clemizole HCl administered to the subject is about 0.5 mg/kg, 1 mg/kg, 1.5 mg/kg, 2 mg/kg, 2.5 mg/kg, 5 mg/kg, 7.5 mg/kg, 10 mg/kg, 12.5 mg/kg, or 15 mg/kg.

14. The liquid pharmaceutical composition for use of claim 12 or 13, wherein the liquid pharmaceutical composition is administered at least every 24 hours.

15. The liquid pharmaceutical composition for use of any of claims 12-14, further comprising administering an anti-epilepsy drug, wherein the administering with the anti-epilepsy drug preferably comprises any one of:
administering the liquid pharmaceutical composition separately from the anti-epilepsy drug,
co-administering the liquid pharmaceutical composition with the anti-epilepsy drug, or
administering the liquid pharmaceutical composition sequentially with the anti-epilepsy drug.

16. The liquid pharmaceutical composition for use of any one of claims 12-15, wherein the epilepsy disorder is a pediatric epilepsy disorder, Dravet syndrome, benign Rolandic epilepsy, frontal lobe epilepsy, infantile spasm, juvenile myoclonic epilepsy (JME), juvenile absence epilepsy, childhood absence epilepsy (e.g., pyknolepsy), febrile seizures, progressive myoclonus epilepsy of Lafora, Lennox-Gastaut syndrome, Landau-Kleffner syndrome, Generalized Epilepsy with Febrile Seizures (GEFS+), Severe Myoclonic Epilepsy of Infancy (SMEI), Benign Neonatal Familial Conversions (BNFC), West Syndrome, Ohtahara Syndrome, early myoclonic encephalopathies, migration partial epilepsy, infantile epileptic encephalopathies, Tuberous Sclerosis Complex (TSC), focal cortical dysplasia, Type I Lissencephaly, Miller-Dieker Syndrome, Angelman's syndrome, Fragile X syndrome, epilepsy in autism spectrum disorder, subcortical band heterotopia, Walker-Warburg syndrome, Alzheimer's disease, posttraumatic epilepsy, progressive myoclonus epilepsies, reflex epilepsy, Rasmussen's syndrome, temporal lobe epilepsy, limbic epilepsy, status epilepticus, abdominal epilepsy, massive bilateral myoclonus catamenial epilepsy, Jaksonian seizure disorder, Unverricht-Lundborg disease, or photosensitive epilepsy.

## Patentansprüche

1. Flüssige pharmazeutische Zusammensetzung, umfassend
Clemizol HCl;
ein Lösungsmittel;
ein Konservierungsmittel; und
Glycerin, wobei
das Konservierungsmittel Kaliumsorbat, Natriummethylparahydroxybenzoat, Natriumethylparahydroxybenzoat, Methylparahydroxybenzoat oder Ethylparahydroxybenzoat umfasst,
wobei das Lösungsmittel einen Citratpuffer umfasst, und wobei der pH der flüssigen pharmazeutischen Zusammensetzung von 4 bis 5 reicht.

2. Flüssige pharmazeutische Zusammensetzung nach Anspruch 1, wobei das Glycerin in einer Konzentration von 10 - 30% (Gew./Gew.) und der Citratpuffer in einer Konzentration von 90 - 70% (Gew./Gew.) ist.

3. Flüssige pharmazeutische Zusammensetzung nach Anspruch 1, wobei die Glycerinkonzentration 20% (Gew./Gew.) ist, und die Citratpufferkonzentration 80% (Gew./Gew.) ist.

4. Flüssige pharmazeutische Zusammensetzung nach einem der voranstehenden Ansprüche,
wobei das Konservierungsmittel Methylparahydroxybenzoat, Kaliumsorbat oder Ethylparahydroxybenzoat umfasst.

5. Flüssige pharmazeutische Zusammensetzung nach einem der voranstehenden Ansprüche, ferner umfassend einen Lösungsvermittler, optional wobei der Lösungsvermittler Tocopherol-Polyethylenglykolsuccinat (TPGS) oder superraffiniertem PEG400 ist.

6. Flüssige pharmazeutische Zusammensetzung nach einem der voranstehenden Ansprüche, ferner umfassend einen Süßstoff und einen Geschmacksmodifikator.

7. Flüssige pharmazeutische Zusammensetzung nach einem der voranstehenden Ansprüche, ferner umfassend ein Antioxidans oder einen Bestandteil mit antioxidativen Eigenschaften.

8. Flüssige pharmazeutische Zusammensetzung nach einem der voranstehenden Ansprüche, wobei das Clemizol HCl in einer Konzentration von 1 mg/mL - 30 mg/ml, bevorzugt 5 mg/mL, in der pharmazeutischen Zusammensetzung ist.

9. Flüssige pharmazeutische Zusammensetzung nach einem der voranstehenden Ansprüche, wobei nach einer Lagerung von bis zu 36 Monaten mindestens eine oder mehrere der Folgenden auftreten:
- keine wesentliche Zunahme von Clemizol HCl-Metaboliten oder verwandten Abbauprodukten,
- eine Menge des Konservierungsmittels bei substantiell 97% oder mehr der ursprünglichen Menge des Konservierungsmittels beibehalten wird,
- keine wesentliche Änderung des pH-Werts der pharmazeutischen Zusammensetzung, oder
- keine wesentliche Änderung der Farbe der pharmazeutischen Zusammensetzung, wobei die Lagerung bei einer Temperatur von 2 - 8 °C, 25 °C oder 40 °C, erfolgt.

10. Flüssige pharmazeutische Zusammensetzung nach einem der voranstehenden Ansprüche, ferner umfassend ein Antiepileptikum.

11. Verfahren zur Herstellung einer flüssigen pharmazeutischen Zusammensetzung zur Behandlung einer Epilepsieerkrankung, umfassend:
Lösen von mindestens einem von einem Konservierungsmittel, Clemizol HCl, einer Vielzahl von Hilfsstoffen und Glycerin in einer Lösung, die mindestens eines von einem Lösungsmittel oder einem Lösungsvermittler umfasst, um die flüssige pharmazeutische Zusammensetzung zu bilden, wobei
das Lösungsmittel ein Citratpuffer ist,
das Clemizol HCl in einer Konzentration von 1 mg/mL bis 30 mg/mL in der flüssigen pharmazeutischen Zusammensetzung ist, und
das mindestens eine Konservierungsmittel mindestens eines von Kaliumsorbat, Natriummethylparahydroxybenzoat, Natriumethylparahydroxybenzoat, Methylparahydroxybenzoat oder Ethylparahydroxybenzoat umfasst,
wobei das Verfahren ferner umfasst
Zugabe des Lösungsmittels, wenn die Auflösung in dem Lösungsvermittler ist, oder Zugabe des Lösungsvermittlers, wenn die Auflösung in dem Lösungsmittel erfolgt, und
Einstellen des pH-Wertes der flüssigen pharmazeutischen Zusammensetzung auf pH 4,5 (+/- 0,5).

12. Flüssige pharmazeutische Zusammensetzung nach einem der voranstehenden Ansprüche zur Verwendung bei der Behandlung eines Subjekts mit einer Epilepsieerkrankung, wobei die Verabreichung einen oder mehrere Wege umfasst, ausgewählt aus oral, sublingual, sublabial, bukkal und transmukosal.

13. Flüssige pharmazeutische Zusammensetzung zur Verwendung nach Anspruch 12, wobei die Dosis an Clemizole-HCl, die dem Subjekt verabreicht wird, etwa 0.5 mq/kq, 1 mq/kq, 1.5 mq/kq, 2 mq/kq, 2.5 mq/kq, 5 mq/kq, 7.5 mq/kq, 10 mq/kq, 12.5 mq/kq, oder 15 mq/kq ist.

14. Flüssige pharmazeutische Zusammensetzung zur Verwendung nach Anspruch 12 oder 13, wobei die Flüssige pharmazeutische Zusammensetzung mindestens alle 24 Stunden verabreicht wird.

15. Flüssige pharmazeutische Zusammensetzung zur Verwendung nach einem der Ansprüche 12-14, ferner umfassend Verabreichen eines Antiepileptikums, wobei die Verabreichung mit dem Antiepileptikum bevorzugt eine der Folgenden umfasst:
Verabreichung der flüssigen pharmazeutischen Zusammensetzung separat von dem Antiepileptikum,
Co-applizieren der flüssigen pharmazeutischen Zusammensetzung mit dem Antiepileptikum oder
die aufeinanderfolgende Verabreichung der flüssigen pharmazeutischen Zusammensetzung mit dem Antiepileptikum.

16. Flüssige pharmazeutische Zusammensetzung zur Verwendung nach einem der Ansprüche 12-15, wobei die Epilepsieerkrankung ein/e pädiatrische Epilepsieerkrankung, Dravet-Syndrom, benigne Rolandische Epilepsie, Frontallappenepilepsie, infantile Spasmen, juvenile myoklonische Epilepsie (JME), juvenile Absence-Epilepsie, Kindheits-Absence-Epilepsie (z. B. PVK-Epilepsie), Fieberkrämpfe, progressive myoklonische Epilepsie nach Lafora, Lennox-Gastaut-Syndrom, Landau-Kleffner-Syndrom, generalisierte Epilepsie mit Fieberkrämpfen (GEFS+), schwere myoklonische Epilepsie im Säuglingsalter (SMEI), benigne neonatale familiäre Konversionen (BNFC), West-Syndrom, Ohtahara-Syndrom, frühe myoklonische Enzephalopathien, migrierende partielle Epilepsie, infantile epileptische Enzephalopathien, tuberöser Sklerose-Komplex (TSC), fokale kortikale Dysplasie, Typ-I-Lissenzephalie, Miller-Dieker-Syndrom, Angelman-Syndrom, Fragiles-X-Syndrom, Epilepsie bei Autismus-Spektrum-Störungen, subkortikale Bandheterotopie, Walker- Warburg-Syndrom, Alzheimer-Krankheit, posttraumatische Epilepsie, progressive Myoklonus-Epilepsien, Reflexepilepsie, Rasmussen-Syndrom, Temporallappenepilepsie, limbische Epilepsie, Status epilepticus, abdominale Epilepsie, massive bilaterale Myoklonus-Katamenialepilepsie, Jackson-Anfallssyndrom, Unverricht-Lundborg-Krankheit oder photosensitive Epilepsie ist.

## Revendications

1. Composition pharmaceutique liquide comprenant
du clémizole HCl ;
un solvant ;
un conservateur ; et
du glycérol, où
le conservateur comprend du sorbate de potassium, du parahydroxybenzoate de méthyle sodique, du parahydroxybenzoate d'éthyle sodique, du parahydroxybenzoate de méthyle ou du parahydroxybenzoate d'éthyle,
où le solvant comprend un tampon citrate, et où
le pH de la composition pharmaceutique liquide est compris entre 4 et 5.

2. Composition pharmaceutique liquide selon la revendication 1, où le glycérol est à une concentration de 10 à 30 % (p/p) et le tampon citrate est à une concentration de 90 à 70 % (p/p).

3. Composition pharmaceutique liquide selon la revendication 1, où la concentration de glycérol est de 20 % (p/p) et la concentration de tampon citrate est de 80 % (p/p).

4. Composition pharmaceutique liquide selon l'une quelconque des revendications précédentes,
où le conservateur comprend du parahydroxybenzoate de méthyle, du sorbate de potassium ou du parahydroxybenzoate d'éthyle.

5. Composition pharmaceutique liquide selon l'une quelconque des revendications précédentes, comprenant en outre un agent solubilisant, facultativement où l'agent solubilisant est du succinate de tocophérol polyéthylène glycol (TPGS) ou du super-refined PEG400.

6. Composition pharmaceutique liquide selon l'une quelconque des revendications précédentes, comprenant en outre un édulcorant et un modificateur de goût.

7. Composition pharmaceutique liquide selon l'une quelconque des revendications précédentes, comprenant en outre un antioxydant ou un composant avec des propriétés antioxydantes.

8. Composition pharmaceutique liquide selon l'une quelconque des revendications précédentes, où le clémizole HCl est à une concentration de 1 mg/ml à 30 mg/ml, de préférence de 5 mg/ml, dans la composition pharmaceutique.

9. Composition pharmaceutique liquide selon l'une quelconque des revendications précédentes, où après un stockage allant jusqu'à 36 mois, au moins l'un quelconque ou plusieurs des événements suivants se produisent :
- il n'y a pas d'augmentation substantielle de métabolites de clémizole HCl ni de produits de dégradation associés,
- une quantité du conservateur est maintenue à pratiquement 97 % ou plus de la quantité initiale du conservateur,
- il n'y a pas de modification substantielle du pH de la composition pharmaceutique,
- il n'y a pas de modification substantielle de la couleur de la composition pharmaceutique, où le stockage se produit à une température de 2 à 8 °C, de 25 °C ou de 40 °C.

10. Composition pharmaceutique liquide selon l'une quelconque des revendications précédentes, comprenant en outre un médicament antiépileptique.

11. Procédé de préparation d'une composition pharmaceutique liquide pour le traitement d'un trouble épileptique, comprenant :
dissoudre au moins un parmi un conservateur, du clémizole HCl, une pluralité d'excipients et du glycérol dans une solution comprenant au moins un parmi un solvant ou un agent solubilisant pour former la composition pharmaceutique liquide, où
le solvant est un tampon citrate,
le clémizole HCl est à une concentration de 1 mg/ml à 30 mg/ml dans la composition pharmaceutique liquide, et
l'au moins un conservateur comprend au moins l'un parmi le sorbate de potassium, le parahydroxybenzoate de méthyle sodique, le parahydroxybenzoate d'éthyle sodique, le parahydroxybenzoate de méthyle ou le parahydroxybenzoate d'éthyle,
où le procédé comprend en outre :
ajouter le solvant lorsque la dissolution se produit dans l'agent solubilisant ou ajouter l'agent solubilisant lorsque la dissolution se produit dans le solvant, et
ajuster le pH de la composition pharmaceutique liquide à un pH 4,5 (+/-0,5).

12. Composition pharmaceutique liquide selon l'une quelconque des revendications précédentes pour utilisation dans le traitement d'un sujet ayant un trouble épileptique, où l'administration comprend une ou plusieurs voies choisies parmi la voie orale, sublinguale, sous-labiale, buccale et transmuqueuse.

13. Composition pharmaceutique liquide pour son utilisation selon la revendication 12, où la dose de clémizole HCl administrée au sujet est d'environ 0,5 mg/kg, 1 mg/kg, 1,5 mg/kg, 2 mg/kg, 2,5 mg/kg, 5 mg/kg, 7,5 mg/kg, 10 mg/kg, 12,5 mg/kg ou 15 mg/kg.

14. Composition pharmaceutique liquide pour son utilisation selon la revendication 12 ou 13, où la composition pharmaceutique liquide est administrée au moins toutes les 24 heures.

15. Composition pharmaceutique liquide pour son utilisation selon l'une quelconque des revendications 12 à 14, comprenant en outre l'administration d'un médicament antiépileptique, où l'administration du médicament antiépileptique comprend de préférence l'un quelconque des événements suivants :
administrer la composition pharmaceutique liquide séparément du médicament antiépileptique,
co-administrer la composition pharmaceutique liquide avec le médicament antiépileptique, ou
administrer la composition pharmaceutique liquide séquentiellement avec le médicament antiépileptique.

16. Composition pharmaceutique liquide pour son utilisation selon l'une quelconque des revendications 12 à 15, où le trouble épileptique est un trouble épileptique pédiatrique, le syndrome de Dravet, l'épilepsie rolandique bénigne, l'épilepsie du lobe frontal, les spasmes infantiles, l'épilepsie myoclonique juvénile (EMJ), l'épilepsie-absence juvénile, l'épilepsie-absence infantile (par exemple, la pycnolepsie), les convulsions fébriles, l'épilepsie Épilepsie myoclonique progressive de Lafora, le syndrome de Lennox-Gastaut, le syndrome de Landau et Kleffner, l'épilepsie généralisée avec convulsions fébriles-plus (GEFS+), l'épilepsie myoclonique sévère du nourrisson (EMSN), les convulsions néonatales familiales bénignes (CNFB), le syndrome de West, le syndrome d'Ohtahara, les encéphalopathies myocloniques précoces, l'épilepsie partielle migrante, les encéphalopathies épileptiques infantiles, la sclérose tubéreuse de Bourneville (STB), la dysplasie corticale focale, la lissencéphalie type 1, le syndrome de Miller-Dieker, le syndrome d'Angelman, le syndrome de l'X fragile, l'épilepsie dans les troubles du spectre autistique, l'hétérotopie en bande sous-corticale, le syndrome de Walker-Warburg, la maladie d'Alzheimer, l'épilepsie post-traumatique, les épilepsies myocloniques progressives, l'épilepsie réflexe, le syndrome de Rasmussen, l'épilepsie du lobe temporal, l'épilepsie limbique, l'état de mal épileptique, l'épilepsie abdominale, l'épilepsie cataméniale à myoclonies massives bilatérales, le syndrome de Jakson, la maladie d'Unverricht-Lundborg ou l'épilepsie photosensible.
